# FASCICULE DE BREVET EUROPEEN

(11) **EP 3 429 611 B1**
(45) Date de publication et mention de la délivrance du brevet: **07.06.2023**
(21) Numéro de dépôt: 17715635.3
(22) Date de dépôt: 14.03.2017
(51) Int. Cl.: A61K 38/16, A61P 25/28

(54) **POLYCOMPLEXES DE COMPOSES POLY-LYSINE POUR LA PREVENTION ET/OU LA LUTTE CONTRE LA SCLEROSE LATERALE AMYOTROPHIQUE**
POLYKOMPLEXE AUS POLYLYSINVERBINDUNGEN ZUR PRÄVENTION UND/ODER BEKÄMPFUNG VON AMYOTROPHER LATERALSKLEROSE
POLYCOMPLEXES OF POLY-LYSINE COMPOUNDS FOR PREVENTING AND/OR COMBATTING AMYOTROPHIC LATERAL SCLEROSIS

(30) Priorité: 14.03.2016 FR 1652093
(43) Date de publication de la demande: 23.01.2019
(73) Titulaire: PLL THERAPEUTICS, 33270 Bouliac (FR)
(72) Inventeur: GEFFARD, Michel, 33400 Talence (FR)
(74) Mandataire: Aquinov
(86) Numéro de dépôt international: PCT/EP2017/056017
(87) Numéro de publication internationale: WO 2017/157952

(56) Documents cités:
- WO-A1-96/15810
- FR-A1- 2 886 153
- FR-A1- 2 905 868

## Description

La présente invention concerne des polycomplexes de composés Poly-lysine (PLL) particuliers ainsi que des compositions les incluant. Ces polycomplexes sont particulièrement utiles comme substances actives d'agents thérapeutiques, en particulier dans la prévention et/ou le traitement de la Sclérose Latérale Amyotrophique dite « SLA ».

La SLA est une maladie neurodégénérative évolutive liée à la mort progressive des motoneurones, cellules nerveuses qui commandent les muscles volontaires. L'atteinte concerne à la fois les motoneurones périphériques, en relation directe avec les muscles, et les motoneurones centraux situés dans le cortex moteur, dans le tronc cérébral, le bulbe et la corne antérieure de la moëlle épinière.

Cette dégénérescence systématisée des motoneurones se traduit par de nombreux troubles moteurs tels que l'existence de spasmes liés à une dysrégulation du tonus musculaire, une augmentation des réflexes ostéo-tendineux, des fasciculations, ou encore des paralysies associées à une atrophie musculaire. Il n'y a pas ou peu d'autres signes d'atteinte neurologique, notamment aucun trouble sensitif, oculomoteur ou démentiel. D'autres symptômes complémentaires se surajoutent aux troubles moteurs, à savoir : constipation, amaigrissement, douleurs, oedèmes, troubles vasomoteurs, troubles du sommeil et respiratoires.

Selon le lieu où débute l'atteinte des motoneurones périphériques, on distingue trois grandes formes de SLA : la forme bulbaire et cervico-brachiale, la forme haute (motoneurones du cortex et centraux) et la forme basse ou lombo-sacrée.

La forme à début spinal est liée à l'atteinte initiale des motoneurones de la moëlle épinière, entraînant des troubles de la motricité des membres supérieurs ou inférieurs.

La forme bulbaire est liée à l'atteinte initiale de neurones moteurs du tronc cérébral et provoque des troubles de la parole et de la déglutition.

Il existe également une forme de SLA qui débute avec l'atteinte des motoneurones du cortex moteur.

Quelle que soit la forme initiale, la neurodégénérescence évolue progressivement en « tache d'huile » vers une forme ayant de multiples handicaps qui influencent le pronostic. Dans la majorité des cas, la mort est due à une déficience respiratoire aggravée par des surinfections bronchiques. La médiane de survie est de 3-5 ans dans plus de 70% des cas.

Quelle que soit la forme initiale, la neurodégénérescence évolue progressivement en « tache d'huile » vers une forme ayant de multiples handicaps qui influencent le pronostic. Dans la majorité des cas, la mort est due à une déficience respiratoire aggravée par des surinfections bronchiques. La médiane de survie est de 3 à 5 ans dans plus de 70% des cas.

Plusieurs gènes (SOD1, ALS2, SPG20, UBQL2, C9ORF72, etc...) ont été identifiés dans des formes familiales de SLA. Mais ces mutations géniques ne représentent que quelques pourcentages de malades atteints de SLA. Dans la majorité des cas la SLA se manifeste de façon sporadique. On ne note pas de gradient de distribution dans le monde comme c'est le cas pour d'autres affections neurologiques comme la Sclérose en Plaques mais toutefois on note des régions (ou isolats) à plus forte prévalence.

Actuellement, il n'existe aucun traitement étiologique capable de stopper l'évolution de la SLA. Le seul produit, le Rilutek ou Riluzole, est un anti-glutamate. Il apporte une médiane de survie de trois à six mois supplémentaires au malade, ce qui est très peu. De plus, ce produit entraîne de nombreux effets secondaires (toxicité hépatique). La prise en charge des malades se limite à la prévention des dysfonctions motrices, à l'aide aux handicaps et aux traitements symptomatiques de la maladie. En outre, cette prise en charge demande l'intervention de professionnels et nécessite une hospitalisation et un suivi particulier, lourd pour les malades et la collectivité.

La recherche de traitements efficaces pour la SLA, capables de maîtriser la progression de la maladie, faciles d'administration, avec peu ou pas d'effets secondaires, est par conséquent un axe prioritaire en santé humaine.

Depuis plus de 20 ans, la SLA a fait l'objet de nombreux travaux fondamentaux en recherche biomédicale, qui ont permis de constater notamment que :
- la neurodégénérescence est liée à une hyperproduction de radicaux libres et d'espèces oxygénées réactives variés, entraînant des modifications protéiques endogènes, une agrégation protéique et la mort neuronale (apoptose) ;
- des "stimuli" extérieurs tels que bactériens, viraux, toxiques, polluants, exercices musculaires intenses, décharges électriques répétées, participent à l'accélération de la neurodégénérescence ;
- lorsque les processus pathogéniques sont engagés dans la SLA, aucune recherche n'a pu identifier des produits ayant une activité inhibitrice, voire stabilisatrice.

Malgré de nombreux essais cliniques, et ce, depuis plus d'une vingtaine d'années, aucun traitement efficace n'existe à ce jour.

L'objectif de l'invention est de proposer une solution thérapeutique dans la prévention et/ou la lutte contre la SLA, plus efficace que les rares traitements thérapeutiques actuels, en particulier une solution capable :
- de maîtriser les mécanismes oxydatifs et radicalaires délétères, et
- de contrôler les "facteurs" ou éléments "extérieurs" (bactériens, viraux, toxiques) intervenant dans la chronicité.

Pour y répondre, l'invention propose d'utiliser des polycomplexes constitués par plusieurs composés Poly-lysine, lesdits composés Poly-lysine étant constitués par au moins une molécule conjuguée à une Poly-lysine. L'invention a pour objet une composition comprenant au moins deux polycomplexes particuliers, chaque polycomplexe étant constitué par plusieurs composés Poly-lysine, lesdits composés Poly-lysine étant constitués par au moins une petite molécule conjuguée à une Poly-lysine :
L'invention a également pour objet ces compositions (comprenant une association de polycomplexes) pour leur utilisation comme substance active d'un médicament, en particulier dans la prévention et/ou le traitement de la SLA.

En effet, de façon avantageuse, les polycomplexes comme substance active d'un médicament selon l'invention sont très efficaces pour prévenir et lutter contre la SLA.

D'autres caractéristiques et avantages ressortiront de la description en détails de l'invention qui va suivre.

L'invention vise donc des compositions comprenant au moins deux polycomplexes constitués par plusieurs composés Poly-lysine, lesdits composés Poly-lysine étant constitués par au moins une petite molécule conjuguée à une Poly-lysine.

Par « polycomplexe » au sens de l'invention, on entend un mélange ou une formulation comprenant plusieurs composés Poly-lysine.

Les polycomplexes selon l'invention comprennent plusieurs composés Poly-lysine. Un composé Poly-lysine est constitué par une ou plusieurs molécules conjuguée(s) à une Poly-lysine.

Par « molécule conjuguée à une Poly-lysine » ou « molécule greffée sur une Poly-lysine », on entend une molécule liée à la Poly-lysine par liaison covalente ou liaison non covalente (notamment hydrogène, ionique Van der Waals).

La poly-lysine est une macromolécule d'unités de lysines, préférentiellement de L-lysine (poly-L-lysine), linéaire ou ramifiée dont le nombre (n) de sous-unités est préférentiellement au moins de 30 lysines : n ≥ 30 lysines.

Les molécules conjuguées à une Poly-lysine dans les composés Poly-lysine sont des « petites molécules », c'est-à-dire des molécules de petite taille, plus particulièrement des molécules de taille inférieure à 1000 daltons, encore plus particulièrement entre 75 et 500 daltons s'il ne s'agit pas de protéines.

Les compositions selon l'invention comprennent au moins deux polycomplexes, chaque polycomplexe étant constitué par plusieurs composés Poly-lysine, lesdits composés Poly-lysine étant constitués par au moins une petite molécule conjuguée à une Poly-lysine.
- au moins un polycomplexe choisi parmi les polycomplexes suivants :
   o un polycomplexe (Polycomplexe 1) constitué exclusivement par les composés Poly-lysine suivants :
      ▪ Cystéine- Anhydride Glutarique -Poly-lysine
      ▪ Cystéine- Glutaraldéhyde réduit -Poly-lysine
      ▪ Taurine- Anhydride Glutarique -Poly-lysine
      ▪ Taurine- Glutaraldéhyde réduit -Poly-lysine
      ▪ Méthionine- Anhydride Glutarique -Poly-lysine
      ▪ Méthionine- Glutaraldéhyde réduit -Poly-lysine
      ▪ Glutathion- Glutaraldéhyde réduit -Poly-lysine
      ▪ Acide thioctique-Poly-lysine ;
         Le Glutaraldéhyde réduit et l'Anhydride glutarique sont des agents réactionnels qui permettent en particulier de lier de façon covalente la petite molécule à la poly-lysine.
   ∘ un polycomplexe (Polycomplexe 2) constitué exclusivement par les composés Poly-lysine suivants :
      ▪ Cystéine- Anhydride Glutarique -Poly-lysine
      ▪ Cystéine- Glutaraldéhyde réduit -Poly-lysine
      ▪ Taurine- Anhydride Glutarique -Poly-lysine
      ▪ Taurine- Glutaraldéhyde réduit -Poly-lysine
      ▪ Méthionine- Anhydride Glutarique -Poly-lysine
      ▪ Méthionine- Glutaraldéhyde réduit -Poly-lysine
      ▪ Glutathion- Glutaraldéhyde réduit -Poly-lysine
      ▪ Acide thioctique-Poly-lysine
      ▪ Biotine-Poly-lysine
      ▪ Acide pantothénique-Poly-lysine
      ▪ Acide ascorbique-Poly-lysine
      ▪ Alpha tocophérol-Poly-lysine
      ▪ Acide rétinoïque-Poly-lysine
      ▪ Coenzyme Q10-Poly-lysine
      ▪ Spermine- Anhydride Glutarique -Poly-lysine.
- **et** au moins un polycomplexe choisi parmi les polycomplexes suivants :
   o Un polycomplexe (Polycomplexe 3) constitué par les composés Poly-lysine suivants :
      ▪ Biotine-Poly-lysine
      ▪ Coenzyme Q10-Poly-lysine
      ▪ Acide rétinoïque-Poly-lysine
      ▪ Acide thioctique-Poly-lysine
      ▪ Acide pantothénique-Poly-lysine
      ▪ Acide ascorbique-Poly-lysine
      ▪ Alpha tocophérol-Poly-lysine
      ▪ Glutathion- Glutaraldéhyde réduit -Poly-lysine
      ▪ Acide oléique-Poly-lysine
   ∘ Un polycomplexe (Polycomplexe 4) constitué par les composés Poly-lysine suivants :
      ▪ Acide oléique-Poly-lysine
      ▪ Acide palmitoléique-Poly-lysine
      ▪ Acide linoléique-Poly-lysine
      ▪ Cholestérol-Poly-lysine
      ▪ Acide laurique-Poly-lysine
      ▪ Acide palmitique-Poly-lysine
      ▪ Acide myristique-Poly-lysine
      ▪ Acide thioctique-Poly-lysine
   ∘ Un polycomplexe (Polycomplexe 5) constitué par les composés Poly-lysine suivants :
      ▪ Acide laurique-Poly-lysine
      ▪ Acide lactique-Poly-lysine
      ▪ Acide pyruvique-Poly-lysine
   ∘ Un polycomplexe (Polycomplexe 6) constitué par les composés Poly-lysine suivants :
      ▪ Acide acétique-Poly-lysine
      ▪ Acide butyrique-Poly-lysine
      ▪ Acide lactique-Poly-lysine
      ▪ Acide propionique-Poly-lysine
      ▪ Acide pyruvique-Poly-lysine
   ∘ un polycomplexe (Polycomplexe 7) constitué par les composés Poly-lysine suivants :
      ▪ Acide rétinoïque-Poly-lysine
      ▪ Alpha tocophérol-Poly-lysine
      ▪ Acide ascorbique-Poly-lysine
      ▪ Coenzyme Q10-Poly-lysine
      ▪ Spermine- Anhydride Glutarique -Poly-lysine
      ▪ Cystéine- Anhydride Glutarique -Poly-lysine
      ▪ Cystéine- Glutaraldéhyde réduit -Poly-lysine
      ▪ Méthionine- Anhydride Glutarique -Poly-lysine
      ▪ Méthionine- Glutaraldéhyde réduit -Poly-lysine
      ▪ Taurine- Anhydride Glutarique -Poly-lysine
      ▪ Taurine- Glutaraldéhyde réduit -Poly-lysine
   ∘ un polycomplexe (Polycomplexe 8) constitué par les composés Poly-lysine suivants :
      ▪ Acide oléique-Poly-lysine
      ▪ Farnésyl cystéine-Poly-lysine
      ▪ Cholestérol-Poly-lysine
      ▪ Acide palmitoléique-Poly-lysine
      ▪ Acide palmitique-Poly-lysine
      ▪ Acide linoléique-Poly-lysine
      ▪ Acide myristique-Poly-lysine
      ▪ Acide thioctique-Poly-lysine
      ▪ Acide azélaïque-Poly-lysine
   ∘ un polycomplexe (Polycomplexe 9) constitué par les composés Poly-lysine suivants :
      ▪ Acide oléique-Poly-lysine
      ▪ Acide thioctique-Poly-lysine
      ▪ Acide rétinoïque-Poly-lysine
      ▪ Coenzyme Q10-Poly-lysine
   ∘ un polycomplexe (Polycomplexe 10) constitué par les composés Poly-lysine suivants :
      ▪ Acide laurique-Poly-lysine
      ▪ Acide oléique-Poly-lysine
      ▪ Acide palmitoléique-Poly-lysine
      ▪ Acide linoléique-Poly-lysine
      ▪ Cholestérol-Poly-lysine
      ▪ Acide palmitique-Poly-lysine
      ▪ Acide myristique-Poly-lysine
      ▪ Acide thioctique-Poly-lysine
      ▪ Coenzyme Q10-Poly-lysine
      ▪ Acide rétinoïque-Poly-lysine
      ▪ Acide pyruvique-Poly-lysine.
      ▪ Glutathion- Glutaraldéhyde réduit -Poly-lysine
      ▪ Cystéine- Anhydride Glutarique -Poly-lysine
      ▪ Cystéine- Glutaraldéhyde réduit -Poly-lysine
      ▪ Taurine- Anhydride Glutarique -Poly-lysine
      ▪ Taurine- Glutaraldéhyde réduit -Poly-lysine
      ▪ Méthionine- Anhydride Glutarique -Poly-lysine
      ▪ Méthionine- Glutaraldéhyde réduit -Poly-lysine
   ∘ un polycomplexe (Polycomplexe 11) constitué par les composés Poly-lysine suivants :
      ▪ Acide oléique-Poly-lysine
      ▪ Acide azélaïque-Poly-lysine
      ▪ Farnésyl cystéine-Poly-lysine
      ▪ Acide thioctique-Poly-lysine
      ▪ Acide palmitique-Poly-lysine
      ▪ Méthionine- Anhydride Glutarique -Poly-lysine
      ▪ Taurine- Anhydride Glutarique -Poly-lysine
      ▪ Acide gamma-aminobutyrique- Glutaraldéhyde réduit -Poly-lysine
   ∘ un polycomplexe (Polycomplexe 12) constitué par les composés Poly-lysine suivants :
      ▪ 5-hydroxytryptamine- Anhydride Glutarique -Poly-lysine
      ▪ 5-hydroxytryptophane- Anhydride Glutarique -Poly-lysine
      ▪ LDopa- Anhydride Glutarique -Poly-lysine
      ▪ Méthionine- Glutaraldéhyde réduit -Poly-lysine
      ▪ Taurine- Glutaraldéhyde réduit -Poly-lysine
      ▪ Acide gamma-aminobutyrique- Glutaraldéhyde réduit -Poly-lysine.

La concentration en chacune des petites molécules conjuguées à la Poly-lysine dans le polycomplexe est préférentiellement comprise entre 6.10⁻⁵ M et 1.10⁻⁴ M, encore plus préférentiellement entre 3.10⁻⁵ M et 2.10⁻⁴ M. Cette concentration peut être mesurée, par exemple, par spectroscopie Ultra-Violet/Visible, par chromatographie en phase gazeuse et en phase liquide couplée à un spectromètre de masse. La corrélation en µg, masse des petites molécules conjuguées à la Poly-lysine, est de 5 à 350 pg/ml.

Les composés Poly-lysine constituant les polycomplexes sont de façon connue obtenus par greffage de petites molécules sur la Poly-lysine. Le procédé de greffage est préférentiellement le suivant :
- la petite molécule est activée par un agent d'activation ou agent de couplage pour former un intermédiaire petite molécule-agent de couplage ;
- pour certaines molécules, comme les acides aminés, cet intermédiaire petite molécule-agent d'activation réagit avec le groupement aminé du résidu lysyl par la formation d'une liaison amide ou d'imine réduite,
   pour d'autres molécules, telle que l'alpha-tocophérol, le groupement carboxylique est directement activé par l'ethylchloroformiate en milieu anhydre ; la liaison amide se fait directement sur le groupement epsilon-aminé de la lysine ;
- ensuite le complexe petite molécule-Poly-lysine est purifié ;
- le produit final est obtenu sous forme liquide ; il peut être lyophilisé afin d'obtenir une forme solide.

Par agent d'activation ou agent de couplage au sens de l'invention, on entend : un produit capable de créer une liaison covalente entre le -COOH ou -NH₂ de la petite molécule et le groupement aminé préférentiellement epsilon aminé du polypeptide. Il peut s'agir par exemple du glutaraldéhyde ou de l'éthyl chloroformiate ou l'anhydride glutarique.

Les polycomplexes selon l'invention sont constitués par plusieurs composés poly-lysine. Ils peuvent se présenter sous forme liquide, ou sous forme solide, c'est-à-dire en poudre après lyophilisation par exemple.

Ils peuvent être obtenus par la mise en oeuvre du procédé suivant :
- chaque composé Poly-lysine (préférentiellement obtenu tel que précédemment décrit) destiné à être intégré dans le polycomplexe, se présente sous forme liquide, et permet après calcul de la concentration en petites molécules, de prendre un volume donné à prélever pour obtenir la concentration finale et le volume final souhaités dans le polycomplexe ;
- avant prélèvement, les composés Poly-Lysine sont agités vigoureusement à la main ou mécaniquement ;
- les composés sont ensuite mélangés; le mélange liquide est réalisé à des températures comprises entre 18 et 22°C, par agitation mécanique vigoureuse, sachant que l'émulsion liquide n'est peu, voire pas visqueuse ; la couleur du mélange liquide varie du blanc laiteux à jaune-orangé laiteux, avec une opacité variable ; l'émulsion mélange liquide est stable d'un point de vue cinétique ;
   Le mélange obtenu peut être notamment congelé puis lyophilisé pour obtenir une poudre. La poudre obtenue peut ensuite être mélangée à des excipients pour obtenir une composition pharmaceutique (préparation thérapeutique, médicament) adaptée en fonction du mode d'application.

En effet, les polycomplexes selon l'invention sont destinés à être administrés à l'animal ou à l'être humain. Ils sont utilisés comme substances actives d'un médicament et sont préférentiellement intégrés dans une composition afin de constituer une préparation thérapeutique ou un médicament.

Selon une variante préférée, la composition comprend au moins les polycomplexes suivants :
- au moins un polycomplexe choisi parmi le Polycomplexe 1 et le Polycomplexe 2, éventuellement au moins le Polycomplexe 1 et le Polycomplexe 2,
- et au moins le Polycomplexe 3.

Selon une autre variante préférée, la composition comprend au moins les polycomplexes suivants :
- au moins un polycomplexe choisi parmi le Polycomplexe 1 et le Polycomplexe 2, éventuellement au moins le Polycomplexe 1 et le Polycomplexe 2,
- et au moins le Polycomplexe 3, le Polycomplexe 4, le Polycomplexe 5 et le Polycomplexe 6.

Pour un choix thérapeutique judicieux, le ou les polycomplexe(s) contenant des anti-oxydants et/ou des piégeurs à radicaux libres sont préférentiellement présents dans la composition en une quantité au moins 2,5 fois plus importante en concentration molaire par rapport aux autres polycomplexes ne contenant pas d'anti-oxydants et/ou de piégeurs à radicaux libres. L'objectif est d'administrer au moins 2,5 fois plus d'antioxydants et piégeurs à radicaux libres que d'acides gras ou autres composés (dérivés lipophiles, acides aminés et dérivés).

En particulier, dans la variante comprenant au moins le Polycomplexe 1 et/ou 2, et les Polycomplexes 3, 4, 5 et 6, la quantité de Polycomplexes 1 et/ou 2 et 3 ensemble (ces polycomplexes comprenant des antioxydants et/ou piégeurs à radicaux libres), est au moins 2,5 fois plus importante que la quantité de Polycomplexes 4, 5 et 6 ensemble.

La concentration en chacune des petites molécules dans les polycomplexes présents est préférentiellement comprise entre 6.10⁻⁵ et 1.10⁻⁴ mol/L, encore plus préférentiellement entre 3.10⁻⁵ et 2.10⁻⁴ mol/L.

Les compositions selon l'invention peuvent se présenter sous toute forme adaptée à une administration thérapeutique à l'homme ou l'animal, en particulier sous forme de comprimés sublinguaux, de gélules, de solution buvable, solution injectable, inhalateur pressurisé à valve doseuse, comprimé, gélule, pommade, crème, suppositoire ou patchs adaptés à une administration par voie per cutanée. Préférentiellement, les compositions selon l'invention sont sous forme de gélules ou comprimés gastro résistantes adaptées à la voie sublinguale, préférentiellement des comprimés sublinguaux.

Les polycomplexes présents dans les compositions sont des substances actives qui présentent un effet thérapeutique.

En plus des polycomplexes selon l'invention, les compositions peuvent comprendre également d'autres constituants, notamment des excipients n'ayant pas d'effet thérapeutique, comme par exemple du mannitol, de l'amidon, de la cellulose microcristalline, du polyéthylèneglycol, du talc, du stéarate de magnésium, de la silice et autres excipients nécessaires à la formulation galénique du comprimé. Si la composition se présente sous une forme adaptée à la voie injectable, elle comprend préférentiellement le sérum isotonique salé. Etant donné que les polycomplexes selon l'invention présentent un effet thérapeutique, l'invention a donc également pour objet ces polycomplexes et/ou compositions pour leurs applications thérapeutiques préventives et/ou curatives, en particulier pour la prévention et/ou le traitement de la SLA.

En particulier l'invention vise les compositions selon l'invention pour leur application comme médicament.

Selon une variante préférée, l'invention vise une composition comprenant un Polycomplexe 1 et/ou 2, pour son utilisation comme médicament en association avec au moins une composition comprenant au moins un Polycomplexe 3.

Selon une autre variante préférée, l'invention vise une composition comprenant un Polycomplexe 1 et/ou 2 (ou une composition comprenant le Polycomplexe 1 et une composition comprenant le Polycomplexe 2) pour son utilisation comme médicament en association avec au moins le Polycomplexe 3, le Polycomplexe 4, le Polycomplexe 5 et le Polycomplexe 6, lesdits polycomplexes 3, 4, 5 et 6 étant formulés dans une même composition ou dans plusieurs compositions différentes.

Pour un choix thérapeutique judicieux, le ou les polycomplexe(s) contenant des anti-oxydants et/ou des piégeurs à radicaux libres sont préférentiellement utilisés en une quantité au moins 2,5 fois plus importante en concentration molaire par rapport aux autres polycomplexes ne contenant pas d'anti-oxydants et/ou de piégeurs à radicaux libres. L'objectif est d'administrer au moins 2,5 fois plus d'antioxydants et piégeurs à radicaux libres que d'acides gras, dérivés et/ou acides aminés et dérivés.

En particulier, dans la variante consistant à administrer au moins le Polycomplexe 1 et/ou 2, et les Polycomplexes 3, 4, 5 et 6, la quantité de Polycomplexes 1 et/ou 2 et 3 ensemble (ces polycomplexes comprenant des antioxydants et/ou piégeurs à radicaux libres), est au moins 2,5 fois plus importante que la quantité de Polycomplexes 4, 5 et 6 ensemble.

La concentration en chacune des petites molécules dans les polycomplexes, est préférentiellement comprise entre 6.10⁻⁵ et 1.10⁻⁴ mol/L, encore plus préférentiellement entre 3.10⁻⁵ et 2.10⁻⁴ mol/L.

Les polycomplexes et compositions selon l'invention sont particulièrement efficaces dans le cadre de la prévention et du traitement de la SLA. Ils permettent de contrôler totalement ou en partie la maladie et d'améliorer les scores de l'échelle de progression fonctionnelle de la SLA à la fois sur la parole, la salivation, l'action d'avaler, l'écriture, la capacité à couper sa nourriture et à tenir des ustensiles, la capacité à s'habiller et à se laver, la capacité à se retourner dans le lit et à ajuster les draps, la marche, la capacité à monter les escaliers et la respiration.

Les vitamines, anti-oxydants et acides aminés sur la Poly-lysine contenus dans les polycomplexes selon l'invention, permettent de lutter contre les mécanismes radicalaires à l'origine de la mort neuronale.

La présence d'acides gras à courte, moyenne et longue chaîne liés à la Poly-lysine, donne des activités antibactérienne, antifongique, virucide mais également immunomodulatrice et piégeuse de toxines. Ces composés ont un rôle étiologique sur les facteurs de chronicité.

Préférentiellement les compositions selon l'invention sont sous forme solide (comprimé sublingual, gélule, etc). Chaque comprimé de 100 mg constitue une unité thérapeutique. Le ou les polycomplexes et la ou les compositions est (sont) alors formé(es) et administrée(s) de façon à ce que :
- la prise quotidienne en petites molécules greffées varie de 15 à 90 µg par jour, et
- chaque composé Poly-lysine représente 0,2 à 2,5 mg par unité thérapeutique administrée.

Selon un mode de réalisation particulièrement adapté, la quantité totale en polycomplexes administrés à une personne malade sur une semaine, c'est-à-dire tous les polycomplexes (1 et/ou 2 et les éventuels autres polycomplexes administrés en association au(x) Polycomplexes(s) 1 et/ou 2 dans une même composition ou dans une ou plusieurs autre(s) composition(s)), est supérieure ou égale à 210 mg. Cette quantité est indépendante du poids du patient à qui elle est administrée.

L'invention est à présent illustrée par des exemples de polycomplexes et de compositions médicamenteuses les incluant, ainsi que par des résultats d'essais réalisés sur des patients volontaires atteints de SLA.

### Exemples de polycomplexes selon l'invention

### Procédé de réalisation des polycomplexes

Les polycomplexes des exemples sont obtenus selon le procédé suivant (à partir de Poly-lysines obtenues par un procédé de greffage connu tel que décrit dans la présente demande) :
- récupération des quantités indiquées de chaque composé Poly-lysine sous forme liquide ;
- obtention pour chaque composé Poly-lysine avec son calcul de concentration en petites molécules, d'un volume donné à prélever par rapport à la concentration finale et au volume final souhaités en polycomplexe, à savoir de 1000 à 10 000 ml;
- avant prélèvement, les composés Poly-lysine sont agités manuellement ou mécaniquement ;
- les composés Poly-lysine en phase liquide sont ensuite mélangés après pipetage sous agitation magnétique ;
- l'émulsion du mélange liquide est réalisée à des températures comprises entre 18 et 22°C, par agitation mécanique vigoureuse à 60 tours/min ;
- le mélange obtenu est congelé puis lyophilisé pour obtenir une poudre.

Ces produits ne présentent pas de risque de toxicité ni d'effets secondaires. Les expérimentations animales ont validé l'absence de toxicité et d'effets indésirables. Ils sont destinés à être intégrés dans une composition.

### Exemple 1 : exemple de Polycomplexe 1 selon l'invention

| Composés Poly-L-Lysine | Concentration finale (en mol/L) |
|---|---|
| | En petites molécules greffées |
| CYSTEINE - Anhydride Glutarique - PLL | 1 x10⁻⁴ |
| CYSTEINE - Glutaraldéhyde réduit - PLL | 1 x10⁻⁴ |
| TAURINE - Anhydride Glutarique - PLL | 1 x10⁻⁴ |
| TAURINE - Glutaraldéhyde réduit - PLL | 1 x10⁻⁴ |
| METHIONINE - Anhydride Glutarique - PLL | 1 x10⁻⁴ |
| METHIONINE - Glutaraldéhyde réduit - PLL | 1 x10⁻⁴ |
| GLUTATHION - Glutaraldéhyde réduit - PLL | 1 x10⁻⁴ |
| ACIDE THIOCTIQUE- PLL | 1 x10⁻⁴ |

| | |
|---|---|
| Poids : 2,3 mg/ml (ou comprimé) | |

### Exemple 2 : exemple de Polycomplexe 2 selon l'invention

| Composés Poly-L-Lysine | Concentration finale (en mol/L) |
|---|---|
| | En petites molécules greffées |
| BIOTINE - PLL | 6 x10⁻⁵ |
| ACIDE PANTOTHENIQUE - PLL | 6 x10⁻⁵ |
| ACIDE ASCORBIQUE - PLL | 6 x10⁻⁵ |
| ALPHA TOCOPHEROL - PLL | 6 x10⁻⁵ |
| GLUTATHION - Glutaraldéhyde réduit - PLL | 1 x10⁻⁴ |
| ACIDE THIOCTIQUE - PLL | 1 x10⁻⁴ |
| ACIDE RETINOIQUE - PLL | 6 x10⁻⁵ |
| CO ENZYME Q10 - PLL | 6 x10⁻⁵ |
| SPERMINE Anhydride Glutarique - PLL | 6 x10⁻⁵ |
| CYSTEINE - Anhydride Glutarique - PLL | 6 x10⁻⁵ |
| CYSTEINE - Glutaraldéhyde réduit - PLL | 6 x10⁻⁵ |
| TAURINE - Anhydride Glutarique - PLL | 6 x10⁻⁵ |
| TAURINE - Glutaraldéhyde réduit - PLL | 6 x10⁻⁵ |
| METHIONINE - Anhydride Glutarique - PLL | 6 x10⁻⁵ |
| METHIONINE - Glutaraldéhyde réduit - PLL | 1 x10⁻⁴ |

| | |
|---|---|
| Poids : 2,5 mg/ml (ou comprimé) | |

### Exemples de polycomplexes pouvant être associés aux polycomplexes selon l'invention dans une composition ou lors du traitement

### Procédé de réalisation des polycomplexes

Les polycomplexes des exemples 3 à 12 sont obtenus selon le procédé suivant (à partir de Poly-lysines obtenues par un procédé de greffage connu tel que décrit dans la présente demande) :
- récupération des quantités indiquées de chaque composé Poly-lysine sous forme liquide ;
- obtention pour chaque composé Poly-lysine avec son calcul de concentration en petites molécules, d'un volume donné à prélever par rapport à la concentration finale et au volume final souhaité en polycomplexe, à savoir de 1000 à 10 000 ml;

- avant prélèvement, les composés Poly-lysine sont agités manuellement ou mécaniquement ;
- les composés Poly-lysine en phase liquide sont ensuite mélangés après pipetage sous agitation magnétique ;
- l'émulsion du mélange liquide est réalisée à des températures comprises entre 18 et 22°C, par agitation mécanique vigoureuse à 60 tours/min ;
- le mélange obtenu est congelé puis lyophilisé pour obtenir une poudre.

Ces produits ne présentent pas de risque de toxicité ni d'effets secondaires. Ils sont destinés à être intégrés dans une composition.

### Exemple 3 : exemple de Polycomplexe 3

| Composés Poly-L-Lysine | Concentration finale (en mol/L) |
|---|---|
| | En petites molécules greffées |
| BIOTINE - PLL | 3 x10⁻⁵ |
| COENZYME Q10 - PLL | 3 x10⁻⁵ |
| ACIDE RETINOIQUE - PLL | 3 x10⁻⁵ |
| ACIDE THIOCTIQUE - PLL | 3 x10⁻⁵ |
| ACIDE PANTOTHENIQUE - PLL | 3 x10⁻⁵ |
| ACIDE ASCORBIQUE - PLL | 3 x10⁻⁵ |
| ALPHA-TOCOPHEROL - PLL | 3 x10⁻⁵ |
| GLUTATHION Glutaraldéhyde réduit - PLL | 3 x10⁻⁵ |
| ACIDE OLEIQUE - PLL | 6 x10⁻⁵ |

| | |
|---|---|
| Poids : 0,7 mg/ml (ou comprimé) | |

### Exemple 4 : exemple de Polycomplexe 4

| Composés Poly-L-Lysine | Concentration finale (en mol/L) |
|---|---|
| | En petites molécules greffées |
| ACIDE OLEIQUE - PLL | 3 x10⁻⁵ |
| ACIDE PALMITOLEIQUE - PLL | 3 x10⁻⁵ |
| ACIDE LINOLEIQUE - PLL | 3 x10⁻⁵ |
| CHOLESTEROL - PLL | 3 x10⁻⁵ |
| ACIDE LAURIQUE - PLL | 3 x10⁻⁵ |
| ACIDE PALMITIQUE - PLL | 3 x10⁻⁵ |
| ACIDE MYRISTIQUE - PLL | 3 x10⁻⁵ |
| ACIDE THIOCTIQUE - PLL | 3 x10⁻⁵ |

| | |
|---|---|
| Poids : 0,4 mg/ml (ou comprimé) | |

### Exemple 5 : exemple Polycomplexe 5

| Composés Poly-L-Lysine | Concentration finale (en mol/L) |
|---|---|
| | En petites molécules greffées |
| ACIDE LAURIQUE - PLL | 5 x 10⁻⁴ |
| ACIDE LACTIQUE - PLL | 2 x 10⁻⁴ |
| ACIDE PYRUVIQUE - PLL | 2 x 10⁻⁴ |

| | |
|---|---|
| Poids : 0,8 mg/ml (ou comprimé) | |

### Exemple 6 : exemple Polycomplexe 6

| Composés Poly-L-Lysine | Concentration finale (en mol/L) |
|---|---|
| | En petites molécules greffées |
| ACIDE ACETIQUE - PLL | 3 x10⁻⁵ |
| ACIDE BUTYRIQUE - PLL | 3 x10⁻⁵ |
| ACIDE LACTIQUE - PLL | 3 x10⁻⁵ |
| ACIDE PROPIONIQUE - PLL | 3 x10⁻⁵ |
| ACIDE PYRUVIQUE - PLL | 3 x10⁻⁵ |

| | |
|---|---|
| Poids : 0,04 mg/ml (ou comprimé) | |

### Exemple 7 : exemple de Polycomplexe 7

| Composés Poly-L-Lysine | Concentration finale (en mol/L) |
|---|---|
| | En petites molécules greffées |
| ACIDE RETINOIQUE - PLL | 3 x10⁻⁵ |
| ALPHA-TOCOPHEROL - PLL | 3 x10⁻⁵ |
| ASCORBIC ACID - PLL | 3 x10⁻⁵ |
| CO ENZYME Q10 - PLL | 3 x10⁻⁵ |
| SPERMINE - Anhydride Glutarique - PLL | 3 x10⁻⁵ |
| CYSTEINE - Anhydride Glutarique - PLL | 3 x10⁻⁵ |
| CYSTEINE - Glutaraldéhyde réduit - PLL | 3 x10⁻⁵ |
| TAURINE - Anhydride Glutarique - PLL | 3 x10⁻⁵ |
| TAURINE - Glutaraldéhyde réduit - PLL | 3 x10⁻⁵ |
| METHIONINE - Anhydride Glutarique - PLL | 3 x10⁻⁵ |
| METHIONINE - Glutaraldéhyde réduit - PLL | 3 x10⁻⁵ |

| | |
|---|---|
| Poids : 1 mg/ml (ou comprimé) | |

### Exemple 8 : exemple de Polycomplexe 8

| Composés Poly-L-Lysine | Concentration finale (en mol/L) |
|---|---|
| | En petites molécules greffées |
| ACIDE OLEIQUE - PLL | 1 x10⁻⁴ |
| FARNESYL CYSTEINE - PLL | 1 x10⁻⁴ |
| CHOLESTEROL - PLL | 3 x10⁻⁵ |
| ACIDE PALMITOLEIQUE - PLL | 6 x10⁻⁵ |
| ACIDE LINOLEIQUE - PLL | 6 x10⁻⁵ |
| ACIDE AZELAIQUE - PLL | 6 x10⁻⁵ |
| ACIDE PALMITIQUE - PLL | 6 x10⁻⁵ |
| ACIDE MYRISTIQUE - PLL | 6 x10⁻⁵ |
| ACIDE THIOCTIQUE - PLL | 6 x10⁻⁵ |

| | |
|---|---|
| Poids : 0,9 mg/ml (ou comprimé) | |

### Exemple 9 : exemple de Polycomplexe 9

| Composés Poly-L-Lysine | Concentration finale (en mol/L) |
|---|---|
| | En petites molécules greffées |
| ACIDE OLEIQUE - PLL | 6 x10⁻⁵ |
| ACIDE THIOCTIQUE - PLL | 3 x10⁻⁵ |
| ACIDE RETINOIQUE - PLL | 3 x10⁻⁵ |
| CO ENZYME Q10 - PLL | 3 x10⁻⁵ |

| | |
|---|---|
| Poids : 0,2 mg/ml (ou comprimé) | |

### Exemple 10 : exemple de Polycomplexe 10

| Composés Poly-L-Lysine | Concentration finale (en mol/L) |
|---|---|
| | En petites molécules greffées |
| ACIDE LAURIQUE - PLL | 6 x10⁻⁵ |
| ACIDE OLEIQUE - PLL | 6 x10⁻⁵ |
| CHOLESTEROL - PLL | 6 x10⁻⁵ |
| ACIDE PALMITOLEIQUE - PLL | 6 x10⁻⁵ |
| ACIDE LINOLEIQUE - PLL | 6 x10⁻⁵ |
| ACIDE PALMITIQUE - PLL | 6 x10⁻⁵ |
| ACIDE MYRISTIQUE - PLL | 6 x10⁻⁵ |
| ACIDE THIOCTIQUE - PLL | 6 x10⁻⁵ |
| ACIDE PYRUVIQUE - PLL | 1 x10⁻⁴ |
| CO ENZYME Q10 - PLL | 3 x10⁻⁵ |
| ACIDE RETINOIQUE - PLL | 3 x10⁻⁵ |
| GLUTATHION - Glutaraldéhyde réduit - PLL | 3 x10⁻⁵ |
| CYSTEINE - Anhydride Glutarique - PLL | 3 x10⁻⁵ |
| CYSTEINE - Glutaraldéhyde réduit - PLL | 3 x10⁻⁵ |
| TAURINE - Anhydride Glutarique - PLL | 3 x10⁻⁵ |
| TAURINE - Glutaraldéhyde réduit - PLL | 3 x10⁻⁵ |
| METHIONINE - Anhydride Glutarique - PLL | 3 x10⁻⁵ |
| METHIONINE - Glutaraldéhyde réduit - PLL | 3 x10⁻⁵ |

| | |
|---|---|
| Poids : 2 mg/ml (ou comprimé) | |

### Exemple 11 : exemple de Polycomplexe 11

| Composés Poly-L-Lysine | Concentration finale (en mol/L) |
|---|---|
| | En petites molécules greffées |
| ACIDE OLEIQUE - PLL | 6 x10⁻⁵ |
| ACIDE AZELAIQUE - PLL | 6 x10⁻⁵ |
| FARNESYL CYSTEINE - PLL | 3 x10⁻⁵ |
| ACIDE THIOCTIQUE - PLL | 3 x10⁻⁵ |
| ACIDE PALMITIQUE - PLL | 3 x10⁻⁵ |
| METHIONINE- Anhydride Glutarique - PLL | 3 x10⁻⁵ |
| TAURINE- Anhydride Glutarique - PLL | 3 x10⁻⁵ |
| ACIDE GAMMA-AMINOBUTYRIQUE - Glutaraldéhyde réduit - PLL | 3 x10⁻⁵ |

| | |
|---|---|
| Poids : 0,6 mg/ml (ou comprimé) | |

### Exemple 12 : exemple de Polycomplexe 12

| Composés Poly-L-Lysine | Concentration finale (en mol/L) |
|---|---|
| | En petites molécules greffées |
| 5-HYDROXYTRYPTAMINE - Anhydride Glutarique - PLL | 1 x10⁻⁴ |
| 5-HYDROXYTRYPTOPHANE - Anhydride Glutarique - PLL | 1 x10⁻⁴ |
| LDOPA - Anhydride Glutarique - PLL | 1 x10⁻⁴ |
| METHIONINE - Glutaraldéhyde réduit - PLL | 1 x10⁻⁴ |
| TAURINE - Glutaraldéhyde réduit - PLL | 1 x10⁻⁴ |
| ACIDE GAMMA-AMINOBUTYRIQUE - Glutaraldéhyde réduit - PLL | 1 x10⁻⁴ |

| | |
|---|---|
| Poids : 1,5 mg/ml (ou comprimé) | |

### Exemples de compositions selon l'invention

### Exemple 13 : exemple de composition correspondant à une unité thérapeutique (comprimé sublingual) comprenant le Polycomplexe 1 seul et les excipients

La composition de l'exemple 13 est la suivante :

| **Composants** | **Teneur (%)** |
|---|---|
| Mannitol (pour compression directe) | 58,7 |
| Amidon prégélatinisé | 16 |
| Cellulose microcristalline | 10 |
| PEG | 5 |
| PVP K30 | 2,5 |
| Talc | 2 |
| Stéarate de Magnésium | 1,5 |
| Silice amorphe | 1 |
| Lévilite | 1 |
| **Total excipients** | **97,7** |
| **Polycomplexe 1** | **2,3** |
| **Total** | **100** |

Le pourcentage est donné en poids de matière sèche.

La composition selon l'invention se présente sous forme d'un comprimé sublingual de 100 mg. La poudre contenant les principes actifs et les excipients est mise sous presse pour constituer le comprimé sublingual de sorte qu'il puisse se dissoudre en 3 à 10 mn. Les principes actifs traversent directement la paroi vasculaire sublinguale et ainsi sont distribués par le sang au niveau des lésions dans l'objectif de neutraliser les espèces radicalaires et éviter ainsi les modifications protéiques endogènes, et donc la mort neuronale.

La posologie pour ce comprimé prescrit seul est de 2 comprimés 3 fois par jour, un jour sur deux, ce qui correspond à une administration de 225 mg par mois d'antioxydants et pièges à radicaux libres.

### Exemple 14 : exemple de composition correspondant à une unité thérapeutique (comprimé sublingual) comprenant le Polycomplexe 2 seul avec les excipients

La composition de l'exemple 14 est la suivante :

| **Composants** | **Teneur (%)** |
|---|---|
| Mannitol (pour compression directe) | 57 |
| Amidon prégélatinisé | 16 |
| Cellulose microcristalline | 10 |
| PEG | 5 |
| PVP K30 | 2,5 |
| Talc | 2 |
| Stéarate de Magnésium | 1,5 |
| Silice amorphe | 1 |
| Lévilite | 1 |
| **Total excipients** | **97** |
| **Polycomplexe 2** | **2** |
| **Total** | **100** |

Le pourcentage est donné en poids de matière sèche.

### La composition selon présente sous forme d'un comprimé sublingual de 100 mg

La poudre contenant les principes actifs et les excipients est mise sous presse pour constituer le comprimé sublingual de sorte qu'il puisse se dissoudre en 3 à 10 mn. Les principes actifs traversent directement la paroi vasculaire sublinguale et ainsi sont distribués par le sang au niveau des lésions dans l'objectif de neutraliser les espèces radicalaires et éviter ainsi les modifications protéiques endogènes, et donc la mort neuronale.

La posologie pour ce comprimé prescrit seul est de 2 comprimés 3 fois par jour, deux jours sur trois, soit 276 mg par mois d'anti-oxydants et pièges à radicaux libres.

### Exemple 15 : exemple d'association du Polycomplexe 1 et du Polycomplexe 2

Les compositions de l'exemple 13 et de l'exemple 14 peuvent être utilisées ensemble dans un même traitement pour un effet synergique.

La posologie pour cet exemple est la suivante :

| | |
|---|---|
| Jour 1 : | Polycomplexe 1 (composition de l'exemple 13) : 2 comprimés, 3 fois par jour, |
| Jour 2 : | Polycomplexe 2 (composition de l'exemple 14) : 2 comprimés 3 fois par jour, |
| Jour 3 : | rien |
| Jour 4 : | même posologie que Jour 1 |
| Jour 5 : | même posologie que Jour 2 |
| Jour 6 : | même posologie que Jour 3 |
| Etc. | |

La quantité d'anti-oxydants et de piégeurs à radicaux libres administrée ce qui correspond à une administration de 288 mg par mois.

### Exemple 16 : exemple d'association des Polycomplexes 1 et 2 avec d'autres polycomplexes

Les compositions des exemples 13 et 14 sont utilisées pour cet exemple ainsi que des compositions identiques concernant les excipients mais comprenant à la place du Polycomplexe 1 ou 2, l'un des Polycomplexes 3 à 5 (exemples 3 à 5).

La posologie pour cet exemple 10 est la suivante :

| | |
|---|---|
| Jour 1 : | Polycomplexe 2 (composition exemple 14) : 2 comprimés, 3 fois par jour, |
| Jour 2 : | Polycomplexe 3 dans une composition : 2 comprimés 2 fois par jour, + Polycomplexe 5 dans une composition : 2 comprimés 3 fois par jour, |
| Jour 3 : | Polycomplexe 1 (composition exemple 13) : 2 comprimés 3 fois par jour, |
| Jour 4 : | Polycomplexe 2 (composition exemple 14) : 2 comprimés 3 fois par jour + Polycomplexe 5 : 2 comprimés 3 fois par jour, |
| Jour 5 : | rien, |
| Jour 6 : | même posologie que Jour 1, |
| Jour 7 : | même posologie que Jour 2, |
| Jour 8 : | même posologie que Jour 3, |
| Jour 9 : | même posologie que Jour 4, |
| Jour 10 : | même posologie que Jour 5, |
| Etc. | |

### Total mg par mois :

- De polycomplexe 1 : 82,8 mg
- De polycomplexe 2 : 144 mg par mois
- De polycomplexe 3 : 16,8 mg par mois
- De polycomplexe 5 : 57,6 mg par mois
- Total mg anti-oxydants et piégeurs à radicaux libres = 243,6 mg par mois,
- Total acide gras plus acide aminé = 57,6 mg par mois
- Rapport entre anti-oxydants + piégeurs à radicaux libres/acides gras + acide aminés = 4,23.

### Essais démontrant l'efficacité de l'invention

### Protocole de traitement / évaluation de l'efficacité

Les polycomplexes et compositions selon l'invention sont particulièrement efficaces dans le cadre de la prévention et du traitement de la SLA. Ils permettent de maîtriser la maladie et d'améliorer les scores sur l'échelle de progression fonctionnelle de la SLA à la fois pour les mécanismes moteurs et le langage.

Les protocoles de traitements sont détaillés en début de chacune des études.

Les patients volontaires ont été suivis à leur demande par des praticiens neurologues ou des médecins. L'activité thérapeutique des polycomplexes a été évaluée sur le plan clinique à l'aide du score ALSQ-40. Cet outil validé évalue les capacités fonctionnelles du patient atteint de SLA (Sancho et Boisson, 2006).

Ce score est établi à partir d'une échelle composée de 10 items :
a. Parole
b. Salivation
c. Action d'avaler
d. Ecriture
e. Capacité à couper sa nourriture et à tenir des ustensiles (patients ne nécessitant pas de tube pour être nourris)
e'. Capacité à couper sa nourriture et à tenir des ustensiles (patients devant être nourris avec un tube) ; l'item e. ou e'. est pris en compte.
f. Capacité à s'habiller et se laver
g. Capacité à se retourner dans le lit et à ajuster les draps
h. Marche
i. Capacité à monter des escaliers
j. Respiration

Chaque item varie entre 0 et 4. La valeur obtenue à un temps t de l'évolution donne la fonctionnalité du patient au moment de l'examen clinique du patient. Plus le score est proche de 40 plus les fonctionnalités sont normales.

La référence R correspond à la courbe médiane de référence mondiale d'évolution de la SLA.

La vitesse d'évolution de la maladie correspond à une perte fonctionnelle de -0,769 point/mois.

A la première évaluation du patient on établit le point de départ de la courbe de référence. Cette courbe permet de situer le score du patient (M) au moment de l'examen et de suivre son évolution dans le temps. Elle permet également d'évaluer l'efficacité des thérapies.

**Tableau d'interprétation**

| **Vitesse moyenne individuelle < ou égale à -0,769** | **Vitesse moyenne individuelle comprise entre -0,769 et 0** | **Vitesse moyenne individuelle = 0** | **Vitesse moyenne individuelle > 0** |
|---|---|---|---|
| **Progression de la maladie** | Ralentissement de la maladie | Stabilisation de la maladie | Inversion de l'évolution de la maladie |
| **Pas d'effet thérapeutique du traitement** | Début d'efficacité du traitement | Traitement efficace | Traitement très efficace avec réparation |

### Etude 1

Cette première étude est relative à l'évaluation de l'efficacité du traitement à l'aide de préparations de polycomplexes contenant des composés Poly-lysine sur 13 patients volontaires atteints de SLA, dont 6 patients sous traitement (entre 4 et 16 mois).

L'étude de 6 nouveaux patients a permis de compléter les résultats.

Les durées de traitement à l'aide de préparations de polycomplexes contenant des composés Poly-lysine sont comprises entre 4 mois et 91 mois (7,5 années). Pour pallier cette variabilité, une méthode d'évaluation adaptée a été utilisée, permettant de comparer les patients entre eux et d'évaluer l'efficacité moyenne du traitement à l'aide de préparations de polycomplexes contenant des composés Poly-lysine sur l'ensemble des 19 patients.

La posologie pour cette étude est la suivante (étant entendu que les polycomplexes sont administrés dans des compositions avec les excipients des compositions des exemples 13 et 14 et que les polycomplexes sont ceux des exemples ) :
- Jour 1 :: Polycomplexe 2 : 1 comprimé, 3 fois par jour,
- Jour 2 :: Polycomplexe 7 : 1 comprimé, 3 fois par jour,
+ Polycomplexe 8 : 1 comprimé, 3 fois par jour,
- Jour 3 :: Polycomplexe 2 : 1 comprimé, 3 fois par jour,
- Jour 4 :: Polycomplexe 3 : 2 comprimés, 3 fois par jour,
- Jour 5 :: rien,
- Jour 6 :: même posologie que Jour 1,
- Jour 7 :: même posologie que Jour 2,
- Jour 8 :: même posologie que Jour 3,
- Jour 9 :: même posologie que Jour 4,
- Jour 10 :: même posologie que Jour 5,
Etc.

Le protocole de traitement est présenté dans le tableau suivant :

| **Protocole de traitement** | | | | | | | | | | | | | | | | | |
|---|---|---|---|---|---|---|---|---|---|---|---|---|---|---|---|---|---|
| **Catégorie** | **1** | | | | **1** | | | | **2** | | | | **1** | | | | |
| **Produit** | **Polycomplexe 2** | | | | **Polycomplexe 7** | | | | **Polycomplexe 8** | | | | **Polycomplexe 3** | | | | **Total cp/jour** |
| **mg** | 2,5 | | | | 1 | | | | 0,9 | | | | 0,7 | | | | |
| | M | Midi | S | **Total** | M | Midi | S | **Total** | M | Midi | S | **Total** | M | Midi | S | **Total** | |
| Jour 1 | 1 | 1 | 1 | **3** | - | - | - | **0** | - | - | - | **0** | - | - | - | **0** | **3** |
| Jour 2 | - | - | - | **0** | 1 | 1 | 1 | **3** | 1 | 1 | 1 | **3** | - | - | - | **0** | **6** |
| Jour 3 | 1 | 1 | 1 | **3** | - | - | - | **0** | - | - | - | **0** | - | - | - | **0** | **3** |
| Jour 4 | - | - | - | **0** | - | - | - | **0** | - | - | - | **0** | 2 | 2 | 2 | **6** | **6** |
| Jour 5 | - | - | - | **0** | - | - | - | **0** | - | - | - | **0** | - | - | - | **0** | **0** |
| **Total cp/mois** | **36** | | | | **18** | | | | **18** | | | | **36** | | | | **108** |
| **Total mg/mois** | **72** | | | | **13,5** | | | | **18** | | | | **25,2** | | | | **128,7** |
| | | | | | | | | | | | | **Total AO+Sc (mg)** | | | | | **110,7** |
| | | | | | | | | | | | | **Total AG+AA (mg)** | | | | | **18** |
| | | | | | | | | | | | | **Rapport AO+Sc/AG+AA** | | | | | **6,15** |

| | | | | | | | | | | | | | | | | | |
|---|---|---|---|---|---|---|---|---|---|---|---|---|---|---|---|---|---|
| *Légende : cp = comprimé, M = matin, S = soir, mg* = *milligramme de polycomplexe, AO = Anti-oxydants, AG = Acides gras, AA = Acides aminés, Sc = Scavengers (piégeurs de radicaux libres), catégorie 1 = AO +Sc, catégorie 2* = *AA +AG* | | | | | | | | | | | | | | | | | |

Le score individuel ALSQ-40 (Echelle de progression fonctionnelle de la SLA) présente des fluctuations au cours du temps.

Afin d'évaluer l'effet global du traitement à l'aide de préparations de polycomplexes contenant des composés Poly-lysine pour chaque patient, on détermine la droite d'évolution moyenne individuelle du score ALSQ-40 au cours du temps dont la pente correspondant à la vitesse moyenne individuelle d'évolution du score ALSQ-40. On réalise un ajustement du nuage des points pour le calcul. Cette vitesse moyenne individuelle d'évolution du score ALSQ-40 est le critère d'évaluation retenu pour l'évaluation de l'effet global du traitement à l'aide de préparations de polycomplexes contenant des composés Poly-lysine.

Les résultats sont présentés dans les tableaux ci-dessous :

| **Evolution des capacités fonctionnelles (ALSQ40)** | **Etude sur 13 patients** | **Etude avec 6 nouveaux patients** |
|---|---|---|
| **Aggravation** | 15 % | 17 % |
| **Ralentissement** | 54 % | 33 % |
| **Stabilisation** | 8% | 17 % |
| **Inversion de la maladie = Amélioration** | 23% | 33 % |
| **Taux d'évolution favorable** | 85 % | 83% |

| | | |
|---|---|---|
| *Pourcentage de l'évolution des patients basée sur le score ALSQ40 prenant en compte les capacités fonctionnelles de chaque patient. On note une évolution favorable entre* 83 *et 85%.* | | |

| **Evolution des capacités fonctionnelles (ALSQ40)** | **Etude sur 13 patients** | **Etude avec 6 nouveaux patients** |
|---|---|---|
| Vitesse moyenne du score pour l'ensemble des malades | -0,391 | -0,419 |
| Vitesse moyenne du score référence mondiale | -0,769 point/mois | |
| Taux de ralentissement de la perte des capacités fonctionnelles | 49,15 % | 45,51 % |
| Gain en mois | 50,3 | 43,5 |
| Gain en années | 4,19 | 3,63 |

| | | |
|---|---|---|
| *On note un gain de survie en moyenne de 3,6 à 4,2 années grâce aux préparations de polycomplexes contenant des composés Poly-lysine.* | | |

L'analyse de ces données permet de confirmer l'efficacité de l'invention.

On observe une évolution favorable de l'état clinique dans plus de 83 % des patients traités à l'aide de préparations de polycomplexes contenant des composés Poly-lysine qui se traduit selon les cas, soit par un ralentissement de la perte des capacités fonctionnelles, soit par une stabilisation des capacités fonctionnelles, soit par une amélioration des capacités fonctionnelles

Le gain pour les patients est de 4 ans en moyenne ce qui représente une durée de survie pratiquement doublée par rapport à la référence mondiale.

### Etude 2

Cette étude avait pour but de démontrer l'efficacité de préparations de polycomplexes contenant des composés Poly-lysine chez les patients volontaires atteints de SLA.

La posologie pour cette étude est la suivante (étant entendu que les polycomplexes sont administrés dans des compositions avec les excipients des compositions des exemples 13 et 14 et que les polycomplexes sont ceux des exemples) :

| | |
|---|---|
| Jour 1 : | Polycomplexe 2 : 2 comprimés, 2 fois par jour, |
| Jour 2 : | Polycomplexe 4 : 1comprimé, 3 fois par jour + Polycomplexe 1 : 2comprimés, 2 fois par jour, |
| Jour 3 : | Polycomplexe 9 : 2 comprimés, 3 fois par jour + Polycomplexe 8 : 2comprimés, 3 fois par jour, |
| Jour 4 : | Polycomplexe 4 : 1 comprimé, 3 fois par jour + Polycomplexe 3 : 2comprimés, 3 fois par jour |
| Jour 5 : | rien |
| Jour 6 : | même posologie que Jour 1 |
| Jour 7 : | même posologie que Jour 2 |
| Jour 8 : | même posologie que Jour 3 |
| Jour 9 : | même posologie que Jour 4 |
| Jour 10 | : même posologie que Jour 5 |
| Etc. | |

Le protocole de traitement est présenté dans le tableau suivant :

| **Protocole de traitement** | | | | | | | | | | | | | | | | | | | | | | | | | | |
|---|---|---|---|---|---|---|---|---|---|---|---|---|---|---|---|---|---|---|---|---|---|---|---|---|---|---|
| **Catégorie** | **1** | | | | **2** | | | | **1** | | | | **1** 1 | | | | **1** | | | | | **2** | | | | |
| **Produit** | **Polycomplexe 2** | | | | **Polycomplexe 4** | | | | **Polycomplexe 3** | | | | **Polycomplexe 1** | | | | **Polycomplexe 9** | | | | | **Polycomplexe 8** | | | | **Total cp/jour** |
| mg | 2, 5 | | | | 0 ,4 | | | | 0,7 | | | | 2, 3 | | | | 0,2 | | | | | 0,9 | | | | |
| | M | Midi | S | **Total** | M | Midi | S | **Total** | M | Midi | S | **Total** | M | Midi | S | **Total** | M | Midi | S | **Total** | | M | Midi | S | Total | |
| Jour 1 | 2 | - | 2 | **4** | - | - | - | **0** | - | - | - | **0** | - | - | - | **0** | - | - | - | **0** | | - | - | - | **0** | **4** |
| Jour 2 | - | - | | **0** | 1 | 1 | 1 | **3** | - | - | - | **0** | 2 | - | 2 | **4** | - | - | - | **0** | | - | - | - | **0** | **7** |
| Jour 3 | - | - | | **0** | - | - | - | **0** | - | - | - | **0** | - | - | - | **0** | 2 | 2 | 2 | **6** | | 2 | 2 | 2 | **6** | **12** |
| Jour 4 | - | - | | **0** | 1 | 1 | 1 | **3** | 2 | 2 | 2 | **6** | - | - | - | **0** | - | - | - | **0** | | - | - | - | **0** | **9** |
| Jour 5 | - | - | | **0** | - | - | - | **0** | - | - | - | **0** | - | - | - | **0** | - | - | - | **0** | | - | - | - | **0** | **0** |
| **Total cp/mois** | **24** | | | | **36** | | | | **36** | | | | **24** | | | | **36** | | | | | **36** | | | | **192** |
| **Total mg/mois** | **48** | | | | **14,4** | | | | **25,2** | | | | **55,2** | | | | **7,2** | | | | | **36** | | | | **186** |
| | | | | | | | | | | | | | | | | | | | | | **Total AO+Sc (mg)** | | | | | **135,6** |
| | | | | | | | | | | | | | | | | | | | | | **Total AG+AA (mg)** | | | | | **50,4** |
| | | | | | | | | | | | | | | | | | | | | | **Rapport AO+Sc/AG+AA** | | | | | **2,69** |

| | | | | | | | | | | | | | | | | | | | | | | | | | | |
|---|---|---|---|---|---|---|---|---|---|---|---|---|---|---|---|---|---|---|---|---|---|---|---|---|---|---|
| *Légende : catégorie =, cp = comprimé, M = matin, S* = *soir, mg = milligramme de polycomplexe, AO* = *Antioxydants, AG* = *Acides gras, AA* = *Acides aminés, Sc* = *Scavengers (piégeurs de radicaux libres)* | | | | | | | | | | | | | | | | | | | | | | | | | | |

Cette étude était nationale, multicentrique, non randomisée, non aveugle.

L'unité expérimentale était le patient. Le facteur étudié était le score ALSQ-40.

Deux niveaux du score ALSQ-40 ont été définis et comparés :
- Score-M : score évalué sur chaque patient lors des visites de suivi.
- Score-R : score de référence mondiale concernant l'évolution de la maladie sans traitement.

Les critères analysés et le plan d'analyse statistique sont décrits en suivant.
- Analyse descriptive à l'inclusion dans l'étude :
   1. Date de naissance
   2. Date de diagnostic de SLA
   3. Age au diagnostic de SLA
   4. Date de début de traitement
   5. Délai entre la date de diagnostic de SLA et date de début de traitement
   6. Sexe
   7. Score global ALSQ-40
   8. Délai entre la date d'évaluation du score ALSQ-40 à l'inclusion et la date de début de traitement
   9. Score pour chaque item de l'échelle ALSQ-40

Variables qualitatives : effectif, pourcentage, distribution, minimum, 1^{er} quartile, médiane, 3^{ème} quartile, maximum.

Variable quantitatives : minimum, 1^{er} quartile, médiane, 3^{ème} quartile, maximum, moyenne, écart type, intervalle de confiance à 95%.
- Etude des visites :
   1. Durée de suivi
   2. Nombre de visites
   3. Intervalle entre les visites

Analyse descriptive : effectif, pourcentage, distribution, minimum, 1^{er} quartile, médiane, 3^{ème} quartile, maximum, moyenne, écart type, intervalle de confiance à 95%.
- Etude quantitative du score ALSQ-40 :
   1. Etude globale du score ALSQ-40
   2. Etude globale de l'évolution en % du score d'ALSQ-40
   3. Score item/item de l'échelle ALSQ-40
   4. Etude de l'évolution en % du score d'ALSQ-40 en fonction de chaque item

Analyse descriptive : effectif, pourcentage, distribution, minimum, 1^{er} quartile, médiane, 3^{ème} quartile, maximum, moyenne, écart type, intervalle de confiance à 95%.

Analyse comparative : test t entre les scores finaux M et R. L'égalité des variances a été vérifiée à l'aide du Folded F test.
- Etude qualitative du score ALSQ-40 :
   1. Etude globale de l'évolution du score ALSQ-40
   2. Etude de l'évolution en fonction des items du score d'ALSQ-40

Analyse descriptive : effectif, pourcentage, distribution.

Analyse de l'évolution des données par un modèle de régression linéaire : les paramètres suivants ont été analysés pour les scores M et R : coefficient de corrélation R² et P-value associée, intercept et pente (ax + b).

Comparaison des taux de succès/échecs : test du chi² ou test exact de Fisher selon les effectifs théoriques obtenus en hypothèse unilatérale (%succès > % d'échecs).

Cette méthode sera utilisée pour les deux études suivantes.
- Gestion des données :
   Les données ont été saisies manuellement pour chaque cas dans des fichiers Excel individuels.

La cohérence des données a été vérifiée. Les données ont été transférées sous le logiciel SAS (Institute Inc software version 9) pour analyse statistique.

Le niveau de signification a été fixé à p=0.05. Les conditions d'applications des tests utilisés ont été vérifiées.

### - Résultats

21 cas ont été inclus dans cette étude.

| | **CRITERES** | **DESCRIPTION ET COMMENTAIRE** | **RESULTATS** |
|---|---|---|---|
| | **A- Analyse descriptive à l'inclusion dans l'étude** | | |
| 1. | Date de naissance | Intervalle médian correspondant à 57 % des cas étudiés | 1941 - 1950 |
| 2. | Date de diagnostic de la SLA | Intervalle médian correspond aux 2/3 des cas | 2007 - 2010 |
| 3. | Age au diagnostic de la SLA | Age médian | 57 ans |
| 4. | Date de début de traitement | Intervalle médian correspondant à 48 % des cas étudiés | 2009 - 2010 |
| 5. | Délai entre date de diagnostic de la SLA et date de début de traitement | Délai médian | 15,7 mois |
| 6. | Sexe | % d'hommes | 61,90% |
| 7. | Score global ALSQ-40 | Score médian | 33 |
| | | Score moyen | 30 |
| 8. | Délai entre la date d'évaluation du score ALSQ-40 à l'inclusion et la date de début de traitement | Médiane | -6 jours |
| | | Délai moyen | 45 jours |
| 9. Score pour chaque item de l'échelle ALSQ-40 | | | |
| | a. Parole | Moyenne. | 3,4 |
| | b. Salivation | La distribution de chaque item met en évidence deux familles : Les items A, B, C et J avec une moyenne comprise entre 3 et 4 et les autres items présentent une moyenne comprise entre 2 et 3. | 3,4 |
| | c. Action d'avaler | | 3,5 |
| | d. Ecriture | | 2,8 |
| | e. Capacité à couper sa nourriture | | 2,7 |
| | f. Capacité à s'habiller et se lever | | 2,5 |
| | g. Capacité à se retourner dans le lit | | 2,8 |
| | h. Marche | | 2,5 |
| | i. Capacité à monter des escaliers | | 2,3 |
| | j. Respiration | | 3,2 |

| | **B- Etude des visites** | | |
|---|---|---|---|
| 1. | Durée de suivi | Médiane | 665 mois Soit 1,8 an |
| 2. | Nombre de visites | Nombre médian de visites | 7 |
| 3. | Intervalle entre les visites | Médiane | 60jours |
| | | 77% des intervalles entres les visites | <100 jours |

### Etude quantitative du score ALSQ-40 :

### a. Etude globale du score ALSQ-40

| **Données** | **Score médian des malades à l'inclusion** | **Score des malades à la fin de l'étude M** | **Score médian de référence à la fin de l'étude R** |
|---|---|---|---|
| **Médiane** | 33 | 17 | 13,8 |
| **Moyenne ± écart type** | 30,0±8,5 | 19,5±7,9 | 12,3±9,3 |
| **IC95%** | [26,1 ; 33,8] | [15,8 ; 23,1] | [8,1 ; 16,6] |
| **P value** | | **0,011** | |

| | | | |
|---|---|---|---|
| *Légende : IC95% = Intervalle de confiance à 95%* | | | |

Il a été mis en évidence une différence statistiquement significative (p=0,011) entre les scores moyens des groupes M et R.

Le score moyen du groupe M est de 19,5 comparé au score moyen du groupe R de 12,3.

Par rapport au score moyen relevé à l'inclusion la diminution dans le groupe M est de 35%, dans le groupe R cette diminution est de 59%.

### b. Etude globale de l'évolution du % du score d'ALSQ-40

| **Données** | **Score final des malades M/Score inclusion** | **Score final de référence R /Score inclusion** | **% d'amélioration Groupe M/Groupe R** |
|---|---|---|---|
| **Médiane** | -35,3% | -55,5% | 20,0% |
| **Moyenne ± écart type** | -30,5±28,9% | -60,1±30,0% | 29,7±38,8% |
| **IC95%** | [-43,6 ; -18,3] | [-73,8 ; -46,5] | [12,0 ; 47,3] |
| **P value** | **0,0022** | | |

| | | | |
|---|---|---|---|
| *Légende : IC95% = Intervalle de confiance à 95%* | | | |

La diminution moyenne du score ALSQ-40 dans le groupe M est de 30,5%, dans le groupe R cette diminution est de 60,1%. L'amélioration du groupe M comparé au groupe R est donc de 29,7%.

Cette différence est significative (p=0,0022).

### c. Score item/item de l'échelle ALSQ-40

Il a été fait une analyse statistique de chaque item composant le score ALSQ-40. Le tableau suivant présente les résultats de l'évolution de chaque item dans les groupes M et R.

| **Item** | **Score** | **Médiane** | **Moyenne ± écart-type** | **IC95%** | **P-value** |
|---|---|---|---|---|---|
| **Parole (*)** | inclusion | 4,0 | 3,1±1,2 | [2,6 ; 3,7] | |
| | M | 2,0 | 2,3±1,6 | [1,6 ; 3,0] | **0,047** |
| | R | 1,5 | 1,5±1,0 | [1,0 ; 1,9] | |
| **Salivation (*)** | inclusion | 4,0 | 3,4±0,8 | [3,0 ; 3,7] | |
| | M | 3,0 | 2,8±1,3 | [2,2 ; 3,4] | **0,007** |
| | R | 1,6 | 1,5±1,0 | [1,1 ; 2,0] | |
| **Action d'avaler (*)** | inclusion | 4,0 | 3,5±0,6 | [3,3 ; 3,8] | |
| | M | 3,0 | 3,0±1,0 | [2,5 ; 3,4] | **<0,0001** |
| | R | 1,7 | 1,6±1,0 | [1,2 ; 2,1] | |
| **Ecriture** | inclusion | 3,0 | 2,9±1,0 | [2,4 ; 3,4] | |
| | M | 2,0 | 1,6±1,4 | [1,0; 2,3] | 0,32 |
| | R | 1,3 | 1,3±1,0 | [0,8 ; 1,7] | |
| **Capacité à couper** | inclusion | 3,0 | 2,8±1,3 | [2,2 ; 3,4] | |
| | M | 1,0 | 1,2±1,3 | [0,7 ; 1,8] | 0,91 |
| | R | 1,3 | 1,2±1,0 | [0,7 ; 1,7] | |
| **Capacité à s'habiller** | inclusion | 3,0 | 2,6±1,3 | [2,0 ; 3,2] | |
| | M | 1,0 | 1,0±1,2 | [0,4 ; 1,5] | 0,93 |
| | R | 0,6 | 1,0±1,1 | [0,5 ; 1,5] | |
| **Capacité à se retourner** | inclusion | 3,0 | 3,0±1,3 | [2,4 ; 3,5] | |
| | M | 2,0 | 1,7±1,2 | [1,2 ; 2,3] | 0,20 |
| | R | 1,0 | 1,3±1,1 | [0,8 ; 1,8] | |
| **Marche (*)** | inclusion | 3,0 | 2,5±1,0 | [2,0 ; 3,0] | |
| | M | 2,0 | 1,6±0,8 | [1,2 ; 1,9] | **0,029** |
| | R | 1,3 | 1,0±0,8 | [0,6 ; 1,4] | |
| **Capacité à monter** | inclusion | 3,0 | 2,2±1,3 | [1,6 2,8] | |
| | M | 0,0 | 0,8±1,1 | [0,3 ; 1,3] | 0,88 |
| | R | 0,6 | 0,9±0,9 | [0,5 ; 1,3] | |
| **Respiration (*)** | inclusion | 4,0 | 3,3±1,1 | [2,8 ; 3,8] | |
| | M | 4,0 | 3,2±1,2 | [2,7 ; 3,8] | **<0,0001** |
| | R | 1,6 | 1,5±1,2 | [1,0 ; 2,0] | |

| | | | | | |
|---|---|---|---|---|---|
| **(^{∗}) Items statistiquement significatifs** *Légende : IC95% = Intervalle de confiance à 95%* | | | | | |

Il a été mis en évidence une différence statistiquement significative (p<0,05) entre les scores moyens des groupes M et R pour les items suivants : Parole, salivation, action d'avaler, marche et respiration. Dans chaque cas le score-M moyen est supérieur au score-R moyen.

### d. Etude de l'évolution du score d'ALSQ-40 en fonction de chaque item

Le tableau suivant présente pour chacun des items l'évolution en pourcentage du score ALSQ-40 dans les deux groupes M et R par rapport au score à l'inclusion ainsi que l'évolution en pourcentage entre le groupe M comparé au groupe R.

| **Item** | | **Score final M /Score inclusion** | **Score final R /Score inclusion** | **% d'amélioration Groupe M/Groupe R** | **P value** |
|---|---|---|---|---|---|
| **Parole (^{∗})** | **Moyenne** | -29,0±40,0% | -50,8±28,2% | 21,8±26,3% | **0,048** |
| | **IC95%** | [-47,2 ; -10,7] | [-63,6 ; -37,9] | [9,8 ; 33,8] | |
| | | | | | |
| **Salivation (^{∗})** | **Moyenne** | -18,3±29,7% | -54,5±27,2% | 36,3±37,0% | **0,002** |
| | **IC95%** | [-31,8 ; -4,7] | [-66,9 ; -42,1] | [19,4 ; 53,1] | |
| | | | | | |
| **Action d'avaler (^{∗})** | **Moyenne** | -16,0±26,2% | -53,3±28,2% | 37,3±25,3% | **<0,0001** |
| | **IC95%** | [-27,9 ; -4,1] | [-66,1 ; -40,4] | [25,8 ; 48,8] | |
| | | | | | |
| **Ecriture** | **Moyenne** | -38,9±42,3% | -51,0±32,3% | 12,1±41,7% | **0,30** |
| | **IC95%** | [-58,1 ; -19,6] | [-65,8 ; -36,3] | [-6,8 ; 31,1] | |
| | | | | | |
| **Capacité à couper** | **Moyenne** | -51,5±41,8% | -52,8±32,2% | 1,2±45,5% | **0,91** |
| | **IC95%** | [-70,6 ; -32,5] | [-67,4 ; -38,1] | [-19,5 ; 21,9] | |
| | | | | | |
| **Capacité à s'habiller** | **Moyenne** | -55,0±52,0% | -57,5±34,5% | 2,5±55,6% | **0,86** |
| | **IC95%** | [-78,6 ; -31,3] | [-73,2 ; -41,7] | [-22,8 ; 27,8] | |
| | | | | | |
| **Capacité à se retourner** | **Moyenne** | -31,0±47,1% | -52,0±33,2% | 21,0±50,1% | **0,10** |
| | **IC95%** | [-52,4 ; -9,5] | [-67,1 ; -36,9] | [-1,8 ; 43,9] | |
| | | | | | |
| **Marche (*)** | **Moyenne** | -33,0±26,7% | -54,8±33,2% | 21,8±29,8% | **0,024** |
| | **IC95%** | [-45,1 ; -20,8] | [-69,9 ; -39,7] | [8,2 ; 35,3] | |
| | | | | | |
| **Capacité à monter** | **Moyenne** | -52,3±54,1% | -51,1±35,4% | -1,2±42,5% | **0,93** |
| | **IC95%** | [-77,0 ; -27,7] | [-67,2% ; -35,0] | [-20,6 ; 18,1] | |
| | | | | | |
| **Respiration (*)** | **Moyenne** | 0±24,3% | -53,1±32,8% | 53,2±33,6% | **<0,0001** |
| | **IC95%** | [-11,1 ; 11,1] | [-68,1 ; -38,3] | [37,9 ; 68,5] | |

| | | | | | |
|---|---|---|---|---|---|
| **(*) Items statistiquement significatifs** *Légende : IC95% = Intervalle de confiance à 95%* | | | | | |

### Etude qualitative du score ALSQ-40 :

### 1. Etude globale

Les évolutions des scores M et R sont comparées.

Si la pente du score M est positive le cas est considéré comme « amélioré ».

Si la pente du score M est supérieur à la pente du score R et inférieur à 0 le cas est considéré comme « ralentissement de la maladie ».

Si la pente du score M est inférieure ou égale à la pente du score R le cas est considéré comme « dégradé ».

L'évolution a également été regroupée pour définir deux classes « succès » et « échec ». L'amélioration et le ralentissement définissent la classe « succès », la dégradation constitue la classe « échec ».

| **Evolution des capacités fonctionnelles (ALSQ40)** | **Répartition** | | **Succès/Échec** | | **P-value** |
|---|---|---|---|---|---|
| **Amélioration** | 3 | 14,3% | 16 | 76,2% | **0,0008** |
| **Ralentissement** | 13 | 61,9% | | | |
| **Dégradation** | 5 | 23,8% | 5 | 23,8% | |
| **Taux d'évolution favorable** | **21** | | **21** | | |

### 2. Etude en fonction des items

| **Item** | **Evolution** | **Répartition** | | **Succès/Échec** | | **P-value** |
|---|---|---|---|---|---|---|
| **Parole** | Amélioration | 1 | 4,8% | 11 | 52,4% | **0,50** |
| | Ralentissement | 10 | 47,6% | | | |
| | Dégradation | 10 | 47,6% | 10 | 47,6% | |
| | | | | | | |
| **Salivation** | Amélioration | 1 | 4,8% | 13 | 61,9% | **0,11** |
| | Ralentissement | 12 | 57,1% | | | |
| | Dégradation | 8 | 38,1% | 8 | 38,1% | |
| | | | | | | |
| **Action d'avaler** | Amélioration | 1 | 4,8% | 12 | 57,2% | **0,27** |
| | Ralentissement | 11 | 52,4% | | | |
| | Dégradation | 9 | 42,9% | 9 | 42,9% | |
| | | | | | | |
| **Ecriture** | Amélioration | 0 | 0 | 10 | 47,6% | **0,73** |
| | Ralentissement | 10 | 47,6% | | | |
| | Dégradation | 11 | 52,4% | 11 | 52,4% | |
| | | | | | | |
| **Capacité à couper** | Amélioration | 0 | 0 | 6 | 28,6% | **0,99** |
| | Ralentissement | 6 | 28,6% | | | |
| | Dégradation | 15 | 71,4% | 15 | 71,4% | |
| | | | | | | |
| **Capacité** à **s'habiller** | Amélioration | 1 | 4,8% | 4 | 19,1% | **1,00** |
| | Ralentissement | 3 | 14,3% | | | |
| | Dégradation | 17 | 81,0% | 17 | 81,0% | |
| | | | | | | |
| **Capacité à se retourner** | Amélioration | 2 | 9,6% | 6 | 28,6% | **0,99** |
| | Ralentissement | 4 | 19,0% | | | |
| | Dégradation | 15 | 71,4% | 15 | 71,4% | |
| | | | | | | |
| **Marche** | Amélioration | 0 | 0 | 6 | 28,6% | **0,99** |
| | Ralentissement | 6 | 28,6% | | | |
| | Dégradation | 15 | 71,4% | 15 | 71,4% | |
| | | | | | | |
| **Capacité à monter** | Amélioration | 2 | 9,6% | 6 | 28,6% | **0,99** |
| | Ralentissement | 4 | 19,0% | | | |
| | Dégradation | 15 | 71,4% | 15 | 71,4% | |
| | | | | | | |
| **Respiration** | Amélioration | 4 | 19,0% | 11 | 52,4% | **<0,0001** |
| | Ralentissement | 14 | 66,7% | | | |
| | Dégradation | 3 | 14,3% | 3 | 14,3% | |

Les items Parole, salivation, action d'avaler, respiration présentent un taux de succès supérieur à 50%.

Il a été mis en évidence une différence statistiquement significative entre les pourcentages de succès et d'échec pour l'item « respiration » (p<0,0001).

### - Conclusion de l'étude:

L'objectif était d'analyser les résultats de l'étude rétrospective (21 malades). Cette étude avait pour but de démontrer l'efficacité de préparations de polycomplexes contenant des composés Poly-lysine chez les patients volontaires atteints de SLA.

Le facteur d'efficacité étudié était le score ALSQ-40 : Echelle de progression fonctionnelle de la SLA. Le score évalué sur chaque patient lors des visites de suivi a été comparé au score théorique d'évolution de la maladie sans traitement.

21 cas ont été inclus dans cette étude. L'analyse descriptive à l'inclusion a mis en évidence une variabilité importante des paramètres analysés.

L'étude des visites a mis également en évidence une grande diversité sur la durée de suivi, le nombre de visites ainsi que l'écart entre les visites.

L'étude globale quantitative du score ALSQ-40 a mis en évidence une différence statistiquement significative (p=0,0011) entre les scores moyens finaux des groupes M (malades) et R (référence internationale). Le score moyen du groupe M est de 19,5 comparé au score moyen du groupe R de 12,3.

L'amélioration du groupe M par rapport au groupe R est de 29,7%. Cette différence est significative (p=0,0022).

L'analyse item par item a mis en évidence une différence statistiquement significative (p<0,05) entre les scores moyens des groupes M et R pour les items suivants : Parole, salivation, action d'avaler, marche et respiration. Dans chaque cas le score-M moyen est supérieur au score-R moyen.

L'étude qualitative du score ALSQ-40 a mis en évidence un pourcentage de « succès » statistiquement significatif (p=0,0008). Dans le cas de l'analyse item/par item, les items parole, salivation, action d'avaler, respiration présentent un taux de succès supérieur à 50%. Il a été mis en évidence une différence statistiquement significative entre les pourcentages de succès et d'échec pour l'item « respiration » (p<0,0001).

### Etude 3

Cette étude avait pour but de 2 groupes traités à des moments différents.

Le premier groupe (M1) de 21 cas correspond à l'étude 2 et le deuxième groupe (M2) correspond à 18 autres malades volontaires.

La posologie pour cette étude est la suivante (étant entendu que les polycomplexes sont administrés dans des compositions avec les excipients des compositions des exemples 13 et 14 et que les polycomplexes sont ceux des exemples) :

| | |
|---|---|
| Jour 1 : | Polycomplexe 2 : 2 comprimés, 2 fois par jour, |
| Jour 2 : | Polycomplexe 10 : 2 comprimés, 2 fois par jour + Polycomplexe 3 : 2 comprimés, 2 fois par jour, |
| Jour 3 : | Polycomplexe 1 : 2 comprimés, 2 fois par jour + Polycomplexe 11 : 2 comprimés, 2 fois par jour, |
| Jour 4 : | Polycomplexe 11 : 3 comprimés, 1 fois par jour + Polycomplexe 12 : 3 comprimés, 1 fois par jour, |
| Jour 5 : | rien, |
| Jour 6 : | même posologie que Jour 1, |
| Jour 7 : | même posologie que Jour 2, |
| Jour 8 : | même posologie que Jour 3, |
| Jour 9 : | même posologie que Jour 4, |
| Jour 10 : | même posologie que Jour 5, |
| Etc. | |

Le protocole de traitement est présenté dans le tableau suivant :

| **Protocole de traitement** | | | | | | | | | | | | | | | | | | | | | | | | | |
|---|---|---|---|---|---|---|---|---|---|---|---|---|---|---|---|---|---|---|---|---|---|---|---|---|---|
| **Catégorie** | **1** | | | | **1+2(50%)** | | | | **1** | | | | **1** | | | | **2** | | | | **2** | | | | |
| **Produit** | **Polycomplexe 2** | | | | **Polycomplexe 10** | | | | **Polycomplexe 3** | | | | **Polycomplexe 1** | | | | **Polycomplexe 11** | | | | **Polycomplexe 12** | | | | **Total cp/jour** |
| **mg** | 2.5 | | | | 2 | | | | 0.7 | | | | 2,3 | | | | 0,6 | | | | 1,5 | | | | |
| | M | Midi | S | **Total** | M | Midi | S | **Total** | M | Midi | S | **Total** | M | Midi | S5 | **Total** | M | Midi | S | **Total** | M | Midi | S | **Total** | |
| Jour 1 | 2 | - | 2 | **4** | - | - | - | **0** | - | - | - | **0** | - | - | - | **0** | - | - | - | **0** | - | - | - | **0** | **4** |
| Jour 2 | - | - | - | **0** | 2 | - | 2 | **4** | 2 | - | 2 | **4** | - | - | - | **0** | - | - | - | **0** | - | - | - | **0** | **8** |
| Jour 3 | - | - | - | **0** | - | - | - | **0** | - | - | | **- 0** | 2 | - | 2 | **4** | 2 | - | 2 | **4** | - | - | - | **0** | **8** |
| Jour4 | - | - | - | **0** | - | - | - | **0** | - | - | - | **0** | - | - | - | **0** | - | - | 3 | **3** | 3 | - | - | **3** | **6** |
| Jour 5 | - | - | - | 0 | - | - | - | **0** | - | - | - | **0** | - | - | - | **0** | - | - | - | **0** | - | - | - | **0** | **0** |
| **Total cp par mois** | **24** | | | | **24** | | | | **24** | | | | **24** | | | | **42** | | | | **18** | | | | **156** |
| **Total mg/mois** | **48** | | | | **48** | | | | **16,8** | | | | **55,2** | | | | **21** | | | | **19,62** | | | | **208,62** |
| | | | | | | | | | | | | | | | | | | | | **Total AO+Sc (mg)** | | | | | **144** |
| | | | | | | | | | | | | | | | | | | | | **Total AG+AA (mg)** | | | | | **64,62** |
| | | | | | | | | | | | | | | | | | | | | **Rapport AO+Sc/AG+AA** | | | | | **2,23** |

| | | | | | | | | | | | | | | | | | | | | | | | | | |
|---|---|---|---|---|---|---|---|---|---|---|---|---|---|---|---|---|---|---|---|---|---|---|---|---|---|
| *Légende : cp = comprimé, M = matin, S = soir, mg = milligramme de polycomplexe, AO = Anti-oxydants, AG =* *Acides gras, AA* = *Acides aminés, Sc = Scavengers (piégeurs de radicaux libres), catégorie 1 = AO +Sc, catégorie 2 = AA +AG* | | | | | | | | | | | | | | | | | | | | | | | | | |

### Méthodologie:

Cette étude était nationale, multicentrique, non randomisée, non aveugle.

L'unité expérimentale était le patient.

Le facteur étudié était le score ALSQ-40 (Echelle de progression fonctionnelle de la SLA). Trois scores ALSQ-40 ont été définis et comparés :
- Score-M1 :: score évalué sur chaque patient lors de la deuxième étude.
- Score-M2 :: score évalué sur chaque patient lors de l'étude « complémentaire ».
- Score-R2 :: score de référence de l'étude « complémentaire ».

### Critères analysés et plan d'analyse statistique :

Etude globale des scores ALSQ-40 M1, M2 et R2.
- Analyse descriptive-comparabilité des groupes à l'inclusion dans l'étude (début du traitement) :
   1. Score global ALSQ-40,
   2. Durée du traitement.

Variable quantitatives : minimum, 1^{er} quartile, médiane, 3^{ème} quartile, maximum, moyenne, écart type, intervalle de confiance à 95%.

Analyse comparative : test t entre les scores M1 et M2 et les durées de traitement des deux groupes. L'égalité des variances a été vérifiée à l'aide du Folded F test.
- Etude quantitative du score ALSQ-40 :
   - *Etude globale du score ALSQ-40 M1 M2 :*
      ∘ Analyse descriptive : effectif, pourcentage, distribution, minimum, 1^{er} quartile, médiane, 3^{ème} quartile, maximum, moyenne, écart type, intervalle de confiance à 95%.
      ∘ Analyse comparative : test t entre les scores finaux M1 et M2. L'égalité des variances a été vérifiée à l'aide du Folded F test.
      ∘ Analyse de covariance avec comme co variable :
         - Score ALSQ-40 à l'inclusion
         - Durée du traitement
   - *Evolution du score ALSQ-40 M2*/*R2 :*
      o Analyse descriptive : effectif, pourcentage, distribution, minimum, 1^{er} quartile, médiane, 3^{ème} quartile, maximum, moyenne, écart type, intervalle de confiance à 95%.
      ∘ Analyse comparative : test t entre les scores finaux M2 et R2. L'égalité des variances a été vérifiée à l'aide du Folded F test.

Les données ont été saisies manuellement pour chaque cas dans des fichiers Excel individuels.

La cohérence des données a été vérifiée. Les données ont été transférées sous le logiciel SAS (Institute Inc software version 9) pour analyse statistique.

Le niveau de signification a été fixé à p=0,05. Les conditions d'applications des tests utilisés ont été vérifiées.

### - Résultats

### • Analyse descriptive-comparaison des groupes à l'inclusion dans l'étude :

L'inclusion dans l'étude des différents cas correspond à la date d'évaluation la plus proche de la date de début du traitement.
∘ *Score global ALSQ-40*

| **Données** | **Groupe M1** | **Groupe M2** |
|---|---|---|
| **Nombre de malades** | 21 | 18 |
| **Médiane du score à l'inclusion** | 33,0 | 33,5 |
| **Moyenne ± écart type** | 30,0±8,5 | 32,8±5,6 |
| **IC95%** | [26,1 ; 33,8] | [30,0 ; 35,6] |
| **P-value** | 0,24 | |

| | | |
|---|---|---|
| *Légende : IC95% = Intervalle de confiance à 95%* | | |

Il n'a pas été mis en évidence de différence statistiquement significative entre les scores moyens à l'inclusion entre les deux groupes M1 et M2 (p=0,24).
∘ *Durée du traitement (en jours)*

| **Données** | **Groupe M1** | **Groupe M2** |
|---|---|---|
| **Nombre de malades** | 21 | 18 |
| **Médiane des durées de traitement** | 665 | 645 |
| **Moyenne ± écart type** | 908±798 | 747±505 |
| **IC95%** | [545 ; 1271] | [496 ; 998] |
| **P-value** | 0,47 | |

| | | |
|---|---|---|
| *Légende : IC95% = Intervalle de confiance à 95%* | | |

Il n'a pas été mis en évidence de différence statistiquement significative entre les scores moyens à l'inclusion entre les deux groupes M1 et M2 (p=0,47).
• *Etude quantitative du score ALSQ-40 après la période de traitement :*
∘ *Etude globale du score ALSQ-40*
Les variables de distribution du score ALSQ-40 moyen des groupes M1 et M2 à l'évaluation finale.

| **Données** | **Groupe M1** | **Groupe M2** |
|---|---|---|
| **Nombre de malades** | 21 | 18 |
| **Médiane des scores** à **l'évaluation finale** | 17 | 26 |
| **Moyenne ± écart type** | 19,5±7,9 | 25,4±7,1 |
| **IC95%** | [15,8 ; 23,1] | [21,9 ; 29,0] |
| **P value ajustée sur le score à l'inclusion** | **0,035** | |
| **P value ajustée sur la durée de traitement** | **0,014** | |

| | | |
|---|---|---|
| *Légende : IC95% = Intervalle de confiance à 95%* | | |

Il a été mis en évidence une différence statistiquement significative (p=0,035 ajusté sur le score à l'inclusion et p=0,014 ajusté sur la durée de traitement) entre les scores moyens des groupes M1 et M2. Le score moyen du groupe M1 est de 19,5 comparé au score moyen du groupe M2 de 25,4.
∘ *Etude globale du score ALSQ-40 score M2 versus score R2*
Les variables de distribution du score ALSQ-40 moyen M2 et R2 à l'évaluation finale.

| **Données** | **Score à l'inclusion** | **Score M2 final** | **Score R2 final** |
|---|---|---|---|
| **Nombre de malades** | 18 | 18 | 18 |
| **Médiane des scores** | 33 | 26 | 15.3 |
| **Moyenne ± écart type** | 32,8±5,6 | 25,4±7,1 | 15,6±8,9 |
| **IC95%** | [30,0 ; 35,6] | [21,9 ; 29,0] | [11,1 ; 20,0] |
| **P value** | | **0,0008** | |

| | | | |
|---|---|---|---|
| *Légende : IC95% = Intervalle de confiance à 95%* | | | |

Il a été mis en évidence une différence statistiquement significative (p=0,0008) entre les scores moyens M2 et R2. Le score moyen M2 est de 25,4 comparé au score moyen R2 de 15,6. Par rapport au score moyen relevé à l'inclusion la diminution du score M2 est de 23%, la diminution du score R2 est de 52%.
∘ *Etude de l'évolution du score d'ALSQ-40 M2*/*R2*
Le tableau suivant représente l'évolution en pourcentage du score ALSQ-40 dans les deux groupes M2 et R2 par rapport au score à l'inclusion ainsi que l'évolution en pourcentage entre le groupe M2 comparé au groupe R2.

| **D**onnées | **Score final M2 /Score inclusion** | **Score final R2 /Score inclusion** | **% d'amélioration Groupe M2/Groupe R2** |
|---|---|---|---|
| **Nombre de malades** | 18 | 18 | 18 |
| **Médiane des pourcentages** | -10,0% | -55,0% | 23,7% |
| **Moyenne ± écart type** | -20,3±24,3% | -52,4±28,9% | 32,1±33,8% |
| **IC95%** | [-32,4 ; -8,2] | [-66,8 ; -38,0] | [15,3 ; 48,9] |
| **P value** | **0,0010** | | |

| | | | |
|---|---|---|---|
| *Légende : IC95% = Intervalle de confiance à 95%* | | | |

La diminution moyenne du score M2 est de 20,3%, le score R2 diminue de 52,4%. L'amélioration du score M2 comparé au score R2 est donc de 32,1%. Cette différence est significative (p=0,0010).

### - Conclusion

Cette étude avait pour but de comparer l'efficacité de deux traitements chez les patients volontaires atteints de SLA.

Le facteur d'efficacité étudié était le score ALSQ-40 : Echelle de progression fonctionnelle de la SLA.

39 cas ont été inclus dans cette étude. La comparabilité des groupes n'a pas mis en évidence de différence statistique entre les deux groupes

L'étude globale quantitative du score ALSQ-40 a mis en évidence une différence statistiquement significative (p<0,05) entre les scores moyens finaux des groupes M1 et M2. Le score moyen du groupe M1 est de 19,5 comparé au score moyen du groupe M2 de 25,4. La diminution moyenne du score M2 est de 20,3%, le score R2 diminue de 52,4%. L'amélioration du score M2 comparé au score R2 est de 32,1%. Cette différence est significative (p=0,0010).

### Etude 4

Cette étude avait pour but de démontrer l'efficacité de préparations de polycomplexes contenant des composés Poly-lysine chez 31 patients volontaires atteints de SLA.

La posologie pour cette étude est la suivante (étant entendu que les polycomplexes sont administrés dans des compositions avec les excipients des compositions des exemples 13 et 14 et que les polycomplexes sont ceux des exemples) :

| | |
|---|---|
| Jour 1 : | Polycomplexe 2 : 2 comprimés, 3 fois par jour, |
| Jour 2 : | Polycomplexe 3 : 2 comprimés, 2 fois par jour + Polycomplexe 5 : 2 comprimés, 3 fois par jour, |
| Jour 3 : | Polycomplexe 1 : 2 comprimés, 3 fois par jour, |
| Jour 4 : | Polycomplexe 2 : 2 comprimés, 3 fois par jour + Polycomplexe 5 : 2 comprimés, 3 fois par jour, |
| Jour 5 : | rien, |
| Jour 6 : | même posologie que Jour 1, |
| Jour 7 : | même posologie que Jour 2, |
| Jour 8 : | même posologie que Jour 3, |
| Jour 9 : | même posologie que Jour 4, |
| Jour 10 : | même posologie que Jour 5, |
| Etc. | |

Le protocole de traitement est présenté dans le tableau suivant :

| **Protocole de traitement** | | | | | | | | | | | | | | | | | | | |
|---|---|---|---|---|---|---|---|---|---|---|---|---|---|---|---|---|---|---|---|
| **Catégorie** | **1** | | | | **1** | | | | **1** | | | | | **2** | | | | | |
| **Produit** | **Polycomplexe 2** | | | | **Polycomplexe 3** | | | | **Polycomplexe 1** | | | | | **Polycomplexe 5** | | | | | **Total** |
| **mg** | 2,5 | | | | 0,7 | | | | 2,3 | | | | | 0,8 | | | | | |
| | M | Midi | S | **total** | M | Midi | S | **total** | | M | Midi | S | **total** | | M | Midi | S | **total** | |
| Jour 1 | 2 | 2 | 2 | **6** | - | - | | **0** | | - | - | - | **0** | | - | - | - | **0** | **6** |
| Jour 2 | - | - | - | **0** | 2 | - | 2 | **4** | | - | - | - | **0** | | 2 | 2 | 2 | **6** | **10** |
| Jour 3 | - | - | - | **0** | - | - | | **0** | | 2 | 2 | 2 | **6** | | - | - | - | **0** | **6** |
| Jour 4 | 2 | 2 | 2 | **6** | - | - | | **0** | | - | - | - | **0** | | 2 | 2 | 2 | **6** | **12** |
| Jour 5 | - | - | - | **0** | - | - | | **0** | | - | - | - | **0** | | - | - | - | **0** | 0 |
| **Total cp/mois** | **72** | | | | **24** | | | | **36** | | | | | **72** | | | | | **204** |
| **Total mg/mois** | **144** | | | | **16,8** | | | | **82,8** | | | | | **57,6** | | | | | **301,2** |
| | | | | | | | | | | | | | **Total mg AO +Sc** | | | | | | **243,6** |
| | | | | | | | | | | | | | **Total AG + AA** | | | | | | **57,6** |
| | | | | | | | | | | | | | **Rapport AO** + **Sc/AG+AA** | | | | | | **4,23** |

| | | | | | | | | | | | | | | | | | | | |
|---|---|---|---|---|---|---|---|---|---|---|---|---|---|---|---|---|---|---|---|
| *Légende : cp = comprimé, M = matin, S = soir, mg = milligramme de polycomplexe, AO = Anti-oxydants, AG = Acides gras, AA* = *Acides aminés, Sc = Scavengers (piégeurs de radicaux libres), catégorie 1 = AO +Sc, catégorie 2 = AA +AG* | | | | | | | | | | | | | | | | | | | |

- Méthodologie : identique à celle de l'étude 3
- Critères analysés et plan d'analyse statistique : identiques à ceux de l'étude 3
- Gestion des données : identique à celle de l'étude 3

### - Résultats :

| **CRITERES** | | | | **DESCRIPTION ET COMMENTAIRE** | **RESULTATS** |
|---|---|---|---|---|---|
| | | | **A. Analyse descriptive à l'inclusion dans l'étude** | | |
| 1. Date de naissance | | | | Intervalle médian correspondant à 45 % des cas étudiés | 1941 - 1950 |
| 2. Date de diagnostic de SLA | | | | Intervalle médian correspond à 70% des cas | 2007 - 2010 |
| 3. Age au diagnostic de SLA | | | | Age médian | 59 ans |
| 4. Date de début de traitement | | | | Intervalle médian correspondant à 42 % des cas étudiés | 2011 - 2013 |
| 5. Sexe | | | | % d'hommes | 64,50% |
| **Délai entre date de diagnostic de la SLA et date de début de traitement** | | | | **Délai médian** | **18,3 mois** |
| 6. Score global ALSQ-40 | | | | Score médian | 33 |
| | | | | Score Moyen | 30,50 |
| 7. Délai entre la date d'évaluation du score ALSQ-40 à l'inclusion et la date de début de traitement | | | | Médiane | -10 jours |
| | | | | Délai moyen | -17 jours |
| 8. Score pour chaque item de l'échelle ALSQ-40 | | | | | |
| | a. Parole | | | Moyenne. | 3,5 |
| | b. Salivation | | | La distribution de chaque item met en évidence deux familles : Les items A, B, C et J avec une moyenne comprise entre 3 et 4 et les autres items présentent une moyenne comprise entre 2 et 3. | 3,7 |
| | c. Action d'avaler | | | | 3,6 |
| | d. Ecriture | | | | 2,6 |
| | e. Capacité à couper sa nourriture | | | | 2,8 |
| | f. Capacité à s'habiller et se lever | | | | 2,6 |
| | g. Capacité à se retourner dans le lit | | | | 2,9 |
| | h. Marche | | | | 2,6 |
| | | i. Capacité à monter des escaliers | | | 2,4 |
| | j. Respiration | | | | 3,3 |

| | | | **B. Etude des visites** | | |
|---|---|---|---|---|---|
| | 1. Durée de suivi | | | Médiane | 685 mois |
| | | | | | Soit 1,9 an |
| | 2. Nombre de visites | | | Nombre médian de visites | 8 |
| | 3. Intervalle entre les visites | | | Médiane | 71 jours |
| | | | | 69 % des intervalles entres les visites | <100 jours |

### C. Etude quantitative du score ALSQ-40

### a. Etude globale du score ALSQ-40

| **Données** | **Score médian à l'inclusion des malades groupe M** | **Score médian final groupe M** | **Score médian de référence final R** |
|---|---|---|---|
| **Médiane des scores** | 33 | 18 | 12,3 |
| **Moyenne ± écart type** | 30,6±7,9 | 19,8±9,4 | 11,7±9,6 |
| **IC95%** | [27,7 ; 33,5] | [16,3 ; 23,2] | [8,2 ; 15,2] |
| **P value** | | **0,0013** | |

| | | | |
|---|---|---|---|
| *Légende : IC95% = Intervalle de confiance à 95%* | | | |

Il a été mis en évidence une différence statistiquement significative (p=0,0013) entre les scores moyens des groupes M et R. Le score moyen du groupe M est de 19,8 comparé au score moyen du groupe R de 11,7.

Par rapport au score moyen relevé à l'inclusion la diminution dans le groupe M est de 35%, dans le groupe R cette diminution est de 62%.

### b. Etude de l'évolution du score d'ALSQ-40

| **Données** | **Score final M /Score inclusion** | **Score final R /Score inclusion** | | **% d'amélioration Groupe M/Groupe R** |
|---|---|---|---|---|
| **Médiane des pourcentages** | -28,6% | -61,6% | | 16,3% |
| **Moyenne ± écart type** | -32,0±30,8% | -62,8±29,9% | | 30,8±36,2% |
| **IC95%** | [-43,3 ; -20,7] | [-73,8 ; -51,8] | | [17,5 ; 44,1] |
| **P value** | **0,0002** | | | |

| | | | | |
|---|---|---|---|---|
| *Légende : IC95% = Intervalle de confiance à 95%* | | | | |

La diminution moyenne du score d'ALSQ-40 dans le groupe M est de 32,0%, dans le groupe R cette diminution est de 62,8%. L'amélioration du groupe M comparé au groupe R est donc de 30,8%. Cette différence est significative (p=0,0002).

### D. Evolution de chaque item dans l'échelle ALSQ-40

| **Item** | **Scores M et R à l'inclusion** | **Médiane des scores M et R** | **Moyenne ± écart type** | **IC95%** | **P-value** |
|---|---|---|---|---|---|
| **Parole** | inclusion | 3,0 | 2,9±1,4 | [2,4 ; 3,4] | |
| | M | 2,5 | 2,3±1,7 | [1,7 ; 2,9] | **0,0033** |
| | R | 1,3 | 1,2±1,1 | [0,8 ; 1,6] | |
| **Salivation** | inclusion | 4,0 | 3,3±0,9 | [3,0 ; 3,6] | |
| | M | 3,0 | 2,6±1,4 | [2,1 ; 3,2] | **0,0001** |
| | R | 1,3 | 1,3±1,1 | [1,0 ; 1,7] | |
| **Action d'avaler** | inclusion | 4,0 | 3,4±0,9 | [3,0 ; 3,7] | |
| | M | 3,0 | 2,8±1,3 | [2,4 ; 3,3] | **<0,0001** |
| | R | 1,8 | 1,4±1,1 | [1,0 ; 1,8] | |
| **Ecriture** | inclusion | 3,0 | 2,9±1,0 | [2,5 ; 3,2] | |
| | M | 3,0 | 1,8±1,6 | [1,2 ; 2,4] | **0,038** |
| | R | 0,8 | 1,1±1,1 | [0,7 ; 1,5] | |
| **Capacité à couper** | inclusion | 3,0 | 2,6±1,3 | [2,2 ; 3,1] | |
| | M | 1,0 | 1,5±1,3 | [1,0 ; 2,0] | 0,067 |
| | R | 0,3 | 0,9±1,0 | [0,5 ; 1,3] | |
| **Capacité à s'habiller** | inclusion | 3,0 | 2,6 ±1,2 | [2,1 ; 3,0] | |
| | M | 1,0 | 1,3±1,2 | [0,8 ; 1,7] | 0,31 |
| | R | 0,7 | 1,0±1,1 | [0,6 ; 1,3] | |
| **Capacité à se retourner** | inclusion | 3,0 | 3,0±1,3 | [2,5 ; 3,4] | |
| | M | 2,0 | 2,0±1,5 | [1,4 ; 2,5] | **0,031** |
| | R | 1,0 | 1,2±1,2 | [0,7 ; 1,6] | |
| **Marche** | inclusion | 3,0 | 2,6±0,9 | [2,3 ; 2,9] | |
| | M | 2,0 | 1,7±1,1 | [1,3 ; 2,1] | **0,0061** |
| | R | 0,8 | 1,0±1,0 | [0,6 ; 1,3] | |
| **Capacité à monter** | inclusion | 3,0 | 2,2±1,3 | [1,7 ; 2,7] | |
| | M | 1,0 | 1,1±1,2 | [0,7 ; 1,6] | 0,32 |
| | R | 0,5 | 0,9±1,0 | [0,5 ; 1,2] | |
| **Respiration** | inclusion | 3,0 | 3,1±1,2 | [2,7 ; 3,6] | |
| | M | 3,0 | 2,7±1,4 | [2,2 ; 3,2] | **<0,0001** |
| | R | 1,3 | 1,3±1,2 | [0,9 ; 1,7] | |

| | | | | | |
|---|---|---|---|---|---|
| *Légende : IC95% = Intervalle de confiance à 95%* | | | | | |

Il a été mis en évidence une différence statistiquement significative (p<0,05) entre les scores moyens des groupes M et R pour les items suivants : Parole, salivation, action d'avaler, écriture, capacité à se retourner, marche et respiration. Dans chaque cas le score-M moyen est supérieur au score-R moyen.

### E. Evolution de chaque item au sein d'ALSQ-40

Le tableau ci-dessous présente pour chaque item l'évolution des pourcentages dans les groupes M et R. Le pourcentage est obtenu par le rapport au sein des groupes des scores à l'inclusion et des scores finaux.

| | | **Pourcentages des rapports de scores** | | | |
|---|---|---|---|---|---|
| **Item** | | **Groupe M Scores finaux /Scores à l'inclusion** | **Groupe R Scores finaux /Scores à l'inclusion** | **% d'amélioration Groupe M/Groupe R** | **P value** |
| **Parole (*)** | **Moyenne** | -24,8±39,2% | -55,7±32,5% | 30,9±29,0% | **0,0013** |
| | **IC95%** | [-39,2 ; -10,5] | [-67,6 ; -43,8] | [20,2 ; 41,5] | |
| | | | | | |
| **Salivation (*)** | **Moyenne** | -21,5±35,3% | -60,2±29,2% | 38,7±39,0% | **<0,0001** |
| | **IC95%** | [-34,4 ; -8,6] | [-71,0 ; -49,5] | [24,4 ; 53,1] | |
| | | | | | |
| **Action d'avaler (*)** | **Moyenne** | -16,9±30,4% | -56,6±32,4% | 39,7±33,8% | **<0,0001** |
| | **IC95%** | [-28,1 ; -5,8] | [-68,5 ; -44,7] | [27,3 ; 52,1] | |
| | | | | | |
| **Ecriture** | **Moyenne** | -35,2±48,9% | -59,7±32,9% | 24,4±48,4% | **0,025** |
| | **IC95%** | [-53,1 ; -17,3] | [-71,7 ; -47,6] | [6,7 ; 42,2] | |
| | | | | | |
| **Capacité à couper** | **Moyenne** | -38,8±42,0% | -63,9±33,1% | 25,1±45,2% | **0,011** |
| | **IC95%** | [-54,2 ; -23,4] | [-76,0 ; -51,8] | [8,5 ; 41,7] | |
| | | | | | |
| **Capacité à s'habiller** | **Moyenne** | -44,5±47,9% | -59,9±34,8% | 15,4±53,9% | **0,15** |
| | **IC95%** | [-62,1; -26,9] | [-72,7 ; -47,1] | [-4,4 ; 35,2] | |
| | | | | | |
| **Capacité à se retourner** | **Moyenne** | -27,8±47,0% | -55,9±35,0% | 28,0±45,8% | **0,010** |
| | **IC95%** | [-45,1 ; -10,6] | [-68,7 ; -43,0] | [11,2 ; 44,8] | |
| | | | | | |
| **Marche (*)** | **Moyenne** | -30,1±34,8% | -61,5±33,9% | 31,4±35,4% | **0,0007** |
| | **IC95%** | [-42,9 ; -17,3] | [-73,9 ; -49,0] | [18,4 ; 44,4] | |
| | | | | | |
| **Capacité à monter** | **Moyenne** | -38,4±49,1% | -56,4±37,7% | 17,9±49,7% | **0,11** |
| | **IC95%** | [-56,4 ; -20,4] | [-70,2 ; -42,5] | [-0,3 ; 36,1] | |
| | | | | | |
| **Respiration (*)** | **Moyenne** | -10,2±28,8% | -56,0±34,2% | 45,8±38,1% | **<0,0001** |
| | **IC95%** | [-20,8 ; 0,3] | [-68,6 ; -43,5] | [31,8 ; 59,8] | |

| | | | | | |
|---|---|---|---|---|---|
| **(*) Items statistiquement significatifs** *Légende : IC95% = Intervalle de confiance à 95%* | | | | | |

### F. Etude qualitative des scores ALSQ-40

### a. Etude globale

| **Evolution des capacités fonctionnelles (ALSQ40)** | **Répartition des patients** | | **% Succès/Échec** | | **P-value** |
|---|---|---|---|---|---|
| **Amélioration** | 4 | 12,9% | 26 | 83,9% | **<0,0001** |
| **Ralentissement** | 22 | 71,0% | | | |
| **Dégradation** | 5 | 16,1% | 5 | 16,1% | |
| **Total** | **31** | | **31** | | |

### b. Etude en fonction des items

| **Item** | **Evolution** | **Répartition des patients** | | **% Succès/Échec** | | **P-value** |
|---|---|---|---|---|---|---|
| **Parole** | Amélioration | 1 | 3,2% | 18 | 58,1% | **0,20** |
| | Ralentissement | 17 | 54,8% | | | |
| | Dégradation | 13 | 41,9% | 13 | 41,9% | |
| | | | | | | |
| **Salivation** | Amélioration | 1 | 3,2% | 19 | 61,3% | **0,075** |
| | Ralentissement | 18 | 58,1% | | | |
| | Dégradation | 12 | 38,7% | 12 | 38,7% | |
| | | | | | | |
| **Action d'avaler** | Amélioration | 1 | 3,2% | 19 | 61,3% | **0,075** |
| | Ralentissement | 18 | 58,1% | | | |
| | Dégradation | 12 | 38,7% | 12 | 38,7% | |
| | | | | | | |
| **Ecriture** | Amélioration | 1 | 3,2% | 18 | 58,1% | **0,20** |
| | Ralentissement | 17 | 54,8% | | | |
| | Dégradation | 13 | 41,9% | 13 | 41,9% | |
| | | | | | | |
| **Capacité à couper** | Amélioration | 0 | 0,0% | 14 | 45,2% | **0,45** |
| | Ralentissement | 14 | 45,2% | | | |
| | Dégradation | 17 | 54,8% | 17 | 54,8% | |
| | | | | | | |
| **Capacité à s'habiller** | Amélioration | 2 | 6,5% | 10 | 32,3% | **0,0052** |
| | Ralentissement | 8 | 25,8% | | | |
| | Dégradation | 21 | 67,7% | 17 | 67,7% | |
| | | | | | | |
| **Capacité à se retourner** | Amélioration | 3 | 9,7% | 13 | 41,9% | **0,20** |
| | Ralentissement | 10 | 32,2% | | | |
| | Dégradation | 18 | 58,1% | 18 | 58,1% | |
| | | | | | | |
| **Marche** | Amélioration | 0 | 0,0% | 14 | 45,2% | **0,45** |
| | Ralentissement | 14 | 45,2% | | | |
| | Dégradation | 17 | 54,8% | 17 | 54,8% | |
| | | | | | | |
| **Capacité à monter** | Amélioration | 2 | 6,5% | 13 | 41,9% | **0,20** |
| | Ralentissement | 11 | 35,5% | | | |
| | Dégradation | 18 | 51,8% | 18 | 51,8% | |
| | | | | | | |
| **Respiration** | Amélioration | 3 | 9,7% | 22 | 71,0% | **0,0010** |
| | Ralentissement | 19 | 61,3% | | | |
| | Dégradation | 9 | 29,0% | 9 | 29,0% | |

Les items Parole, salivation, action d'avaler, écriture et respiration présentent un taux de succès supérieur à 50%.

Il a été mis en évidence une différence statistiquement significative entre les pourcentages de succès et d'échec pour les items « capacité à s'habiller » et « respiration » (p<0,05).

### - Conclusion

L'objectif était d'analyser l'efficacité de préparations de polycomplexes contenant des composés Poly-lysine chez les patients volontaires atteints de SLA.

Le facteur d'efficacité étudié était le score ALSQ-40 : Echelle de progression fonctionnelle de la SLA. Le score évalué sur chaque patient lors des visites de suivi a été comparé au score théorique d'évolution de la maladie sans traitement.

31 cas ont été inclus dans cette étude. L'analyse descriptive à l'inclusion a mis en évidence une variabilité importante dans les paramètres analysés.

L'étude des visites a mis également en évidence une grande diversité sur la durée de suivi, le nombre de visites ainsi que l'écart entre les visites.

L'étude globale quantitative du score ALSQ-40 a mis en évidence une différence statistiquement significative (p=0,0013) entre les scores moyens finaux des groupes M et R.

Le score moyen du groupe M est de 19,8 comparé au score moyen du groupe R égal à 11,7.

L'amélioration du groupe M par rapport au groupe R est de 30,8%. Cette différence est significative (p=0,0002).

L'analyse item par item a mis en évidence une différence statistiquement significative (p<0,05) entre les scores moyens des groupes M et R pour les items suivants : Parole, salivation, action d'avaler, écriture, capacité à se retourner, marche et respiration. Dans chaque cas le score-M moyen est supérieur au score-R moyen.

L'étude qualitative du score d'ALSQ-40 a mis en évidence un pourcentage de « succès » statistiquement significatif (p<0,0001). Dans le cas de l'analyse item/par item, les items parole, salivation, action d'avaler, écriture, respiration présentent un taux de succès supérieur à 50%.

Il a été mis en évidence une différence statistiquement significative entre les pourcentages de succès et d'échec pour les items Capacité à s'habiller et Respiration (p<0,05).

## Revendications

1. Composition comprenant au moins deux polycomplexes, chaque polycomplexe étant constitué par plusieurs composés Poly-lysine, lesdits composés Poly-lysine étant constitués par au moins une petite molécule conjuguée à une Poly-lysine, **caractérisée en ce que**.
- au moins un polycomplexe est choisi parmi les polycomplexes suivants :
o un polycomplexe constitué exclusivement par les composés Poly-lysine suivants :
▪ Cystéine- Anhydride Glutarique -Poly-lysine
▪ Cystéine- Glutaraldéhyde réduit -Poly-lysine
▪ Taurine- Anhydride Glutarique -Poly-lysine
▪ Taurine- Glutaraldéhyde réduit -Poly-lysine
▪ Méthionine- Anhydride Glutarique -Poly-lysine
▪ Méthionine- Glutaraldéhyde réduit -Poly-lysine
▪ Glutathion- Glutaraldéhyde réduit -Poly-lysine
▪ Acide thioctique-Poly-lysine.
∘ un polycomplexe constitué exclusivement par les composés Poly-lysine suivants :
▪ Cystéine- Anhydride Glutarique -Poly-lysine
▪ Cystéine- Glutaraldéhyde réduit -Poly-lysine
▪ Taurine- Anhydride Glutarique -Poly-lysine
▪ Taurine- Glutaraldéhyde réduit -Poly-lysine
▪ Méthionine- Anhydride Glutarique -Poly-lysine
▪ Méthionine- Glutaraldéhyde réduit -Poly-lysine
▪ Glutathion- Glutaraldéhyde réduit -Poly-lysine
▪ Acide thioctique-Poly-lysine.
▪ Biotine-Poly-lysine
▪ Acide pantothénique-Poly-lysine
▪ Acide ascorbique-Poly-lysine
▪ Alpha tocophérol-Poly-lysine
▪ Acide rétinoïque-Poly-lysine
▪ Coenzyme Q10-Poly-lysine
▪ Spermine- Anhydride Glutarique -Poly-lysine
- et au moins un polycomplexe est choisi parmi les polycomplexes suivants :
o Un polycomplexe constitué par les composés Poly-lysine suivants :
▪ Biotine-Poly-lysine
▪ Coenzyme Q10-Poly-lysine
▪ Acide rétinoïque-Poly-lysine
▪ Acide thioctique-Poly-lysine
▪ Acide pantothénique-Poly-lysine
▪ Acide ascorbique-Poly-lysine
▪ Alpha tocophérol-Poly-lysine
▪ Glutathion- Glutaraldéhyde réduit -Poly-lysine
▪ Acide oléique-Poly-lysine
o Un polycomplexe constitué par les composés Poly-lysine suivants :
▪ Acide oléique-Poly-lysine
▪ Acide palmitoléique-Poly-lysine
▪ Acide linoléique-Poly-lysine
▪ Cholestérol-Poly-lysine
▪ Acide laurique-Poly-lysine
▪ Acide palmitique-Poly-lysine
▪ Acide myristique-Poly-lysine
▪ Acide thioctique-Poly-lysine
o Un polycomplexe constitué par les composés Poly-lysine suivants :
▪ Acide laurique-Poly-lysine
▪ Acide lactique-Poly-lysine
▪ Acide pyruvique-Poly-lysine
o Un polycomplexe constitué par les composés Poly-lysine suivants :
▪ Acide acétique-Poly-lysine
▪ Acide butyrique-Poly-lysine
▪ Acide lactique-Poly-lysine
▪ Acide propionique-Poly-lysine
▪ Acide pyruvique-Poly-lysine
o un polycomplexe constitué par les composés Poly-lysine suivants :
▪ Acide rétinoïque-Poly-lysine
▪ Alpha tocophérol-Poly-lysine
▪ Acide ascorbique-Poly-lysine
▪ Coenzyme Q10-Poly-lysine
▪ Spermine- Anhydride Glutarique -Poly-lysine
▪ Cystéine- Anhydride Glutarique -Poly-lysine
▪ Cystéine- Glutaraldéhyde réduit -Poly-lysine
▪ Méthionine- Anhydride Glutarique -Poly-lysine
▪ Méthionine- Glutaraldéhyde réduit -Poly-lysine
▪ Taurine- Anhydride Glutarique -Poly-lysine
▪ Taurine- Glutaraldéhyde réduit -Poly-lysine
o un polycomplexe constitué par les composés Poly-lysine suivants :
▪ Acide oléique-Poly-lysine
▪ Farnésyl cystéine-Poly-lysine
▪ Cholestérol-Poly-lysine
▪ Acide palmitoléique-Poly-lysine
▪ Acide palmitique-Poly-lysine
▪ Acide linoléique-Poly-lysine
▪ Acide myristique-Poly-lysine
▪ Acide thioctique-Poly-lysine
▪ Acide azélaïque-Poly-lysine
o un polycomplexe constitué par les composés Poly-lysine suivants :
▪ Acide oléique-Poly-lysine
▪ Acide thioctique-Poly-lysine
▪ Acide rétinoïque-Poly-lysine
▪ Coenzyme Q10-Poly-lysine
o un polycomplexe constitué par les composés Poly-lysine suivants :
▪ Acide laurique-Poly-lysine
▪ Acide oléique-Poly-lysine
▪ Acide palmitoléique-Poly-lysine
▪ Acide linoléique-Poly-lysine
▪ Cholestérol-Poly-lysine
▪ Acide palmitique-Poly-lysine
▪ Acide myristique-Poly-lysine
▪ Acide thioctique-Poly-lysine
▪ Coenzyme Q10-Poly-lysine
▪ Acide rétinoïque-Poly-lysine
▪ Acide pyruvique-Poly-lysine.
▪ Glutathion- Glutaraldéhyde réduit -Poly-lysine
▪ Cystéine- Anhydride Glutarique -Poly-lysine
▪ Cystéine- Glutaraldéhyde réduit -Poly-lysine
▪ Taurine- Anhydride Glutarique -Poly-lysine
▪ Taurine- Glutaraldéhyde réduit -Poly-lysine
▪ Méthionine- Anhydride Glutarique -Poly-lysine
▪ Méthionine- Glutaraldéhyde réduit -Poly-lysine
o un polycomplexe constitué par les composés Poly-lysine suivants :
▪ Acide oléique-Poly-lysine
▪ Acide azélaïque-Poly-lysine
▪ Farnésyl cystéine-Poly-lysine
▪ Acide thioctique-Poly-lysine
▪ Acide palmitique-Poly-lysine
▪ Méthionine- Anhydride Glutarique -Poly-lysine
▪ Taurine- Anhydride Glutarique -Poly-lysine
▪ Acide gamma-aminobutyrique- Glutaraldéhyde réduit -Poly-lysine
o un polycomplexe constitué par les composés Poly-lysine suivants :
▪ 5-hydroxytryptamine- Anhydride Glutarique -Poly-lysine
▪ 5-hydroxytryptophane- Anhydride Glutarique -Poly-lysine
▪ LDopa- Anhydride Glutarique -Poly-lysine
▪ Méthionine- Glutaraldéhyde réduit -Poly-lysine
▪ Taurine- Glutaraldéhyde réduit -Poly-lysine
▪ Acide gamma-aminobutyrique- Glutaraldéhyde réduit -Poly-lysine.

2. Composition selon la précédente revendication, **caractérisée en ce que** le ou les polycomplexe(s) comprenant des antioxydants et/ou des piégeurs à radicaux libres sont présents en une quantité au moins 2,5 fois plus importante en poids que les autres polycomplexes.

3. Composition selon l'une des précédentes revendications, **caractérisée en ce que** la concentration en chacune desdites petites molécules dans chaque polycomplexe est comprise entre 6.10⁻⁵ M et 1.10⁻⁴ M.

4. Composition selon l'une des précédentes revendications-pour son utilisation comme médicament.

5. Composition comprenant au moins un polycomplexe, constitué exclusivement par les composés Poly-lysine suivants :
o Cystéine- Anhydride Glutarique -Poly-lysine
o Cystéine- Glutaraldéhyde réduit -Poly-lysine
o Taurine- Anhydride Glutarique -Poly-lysine
o Taurine- Glutaraldéhyde réduit -Poly-lysine
o Méthionine- Anhydride Glutarique -Poly-lysine
o Méthionine- Glutaraldéhyde réduit -Poly-lysine
o Glutathion- Glutaraldéhyde réduit -Poly-lysine
o Acide thioctique-Poly-lysine,
pour son utilisation comme médicament en association avec au moins une composition comprenant au moins un polycomplexe choisi parmi les polycomplexes suivants :
o Un polycomplexe constitué par les composés Poly-lysine suivants :
▪ Biotine-Poly-lysine
▪ Coenzyme Q10-Poly-lysine
▪ Acide rétinoïque-Poly-lysine
▪ Acide thioctique-Poly-lysine
▪ Acide pantothénique-Poly-lysine
▪ Acide ascorbique-Poly-lysine
▪ Alpha tocophérol-Poly-lysine
▪ Glutathion- Glutaraldéhyde réduit -Poly-lysine
▪ Acide oléique-Poly-lysine
o Un polycomplexe constitué par les composés Poly-lysine suivants :
▪ Acide oléique-Poly-lysine
▪ Acide palmitoléique-Poly-lysine
▪ Acide linoléique-Poly-lysine
▪ Cholestérol-Poly-lysine
▪ Acide laurique-Poly-lysine
▪ Acide palmitique-Poly-lysine
▪ Acide myristique-Poly-lysine
▪ Acide thioctique-Poly-lysine
o Un polycomplexe constitué par les composés Poly-lysine suivants :
▪ Acide laurique-Poly-lysine
▪ Acide lactique-Poly-lysine
▪ Acide pyruvique-Poly-lysine
o Un polycomplexe constitué par les composés Poly-lysine suivants :
▪ Acide acétique-Poly-lysine
▪ Acide butyrique-Poly-lysine
▪ Acide lactique-Poly-lysine
▪ Acide propionique-Poly-lysine
▪ Acide pyruvique-Poly-lysine
o un polycomplexe constitué par les composés Poly-lysine suivants :
▪ Acide rétinoïque-Poly-lysine
▪ Alpha tocophérol-Poly-lysine
▪ Acide ascorbique-Poly-lysine
▪ Coenzyme Q10-Poly-lysine
▪ Spermine- Anhydride Glutarique -Poly-lysine.
▪ Cystéine- Anhydride Glutarique -Poly-lysine
▪ Cystéine- Glutaraldéhyde réduit -Poly-lysine
▪ Méthionine- Anhydride Glutarique -Poly-lysine
▪ Méthionine- Glutaraldéhyde réduit -Poly-lysine
▪ Taurine- Anhydride Glutarique -Poly-lysine
▪ Taurine- Glutaraldéhyde réduit -Poly-lysine
o un polycomplexe constitué par les composés Poly-lysine suivants :
▪ Acide oléique-Poly-lysine
▪ Farnésyl cystéine-Poly-lysine
▪ Cholestérol-Poly-lysine
▪ Acide palmitoléique-Poly-lysine
▪ Acide palmitique-Poly-lysine
▪ Acide linoléique-Poly-lysine
▪ Acide myristique-Poly-lysine
▪ Acide thioctique-Poly-lysine
▪ Acide azélaïque-Poly-lysine
o un polycomplexe constitué par les composés Poly-lysine suivants :
▪ Acide oléique-Poly-lysine
▪ Acide thioctique-Poly-lysine
▪ Acide rétinoïque-Poly-lysine
▪ Coenzyme Q10-Poly-lysine.
chaque polycomplexe étant constitué par plusieurs composés Poly-lysine, lesdits composés Poly-lysine étant constitués par au moins une petite molécule conjuguée à une Poly-lysine.

6. Composition comprenant au moins un polycomplexe, constitué exclusivement par les composés Poly-lysine suivants :
∘ Cystéine- Anhydride Glutarique -Poly-lysine
∘ Cystéine- Glutaraldéhyde réduit -Poly-lysine
∘ Taurine- Anhydride Glutarique -Poly-lysine
∘ Taurine- Glutaraldéhyde réduit -Poly-lysine
∘ Méthionine- Anhydride Glutarique -Poly-lysine
∘ Méthionine- Glutaraldéhyde réduit -Poly-lysine
∘ Glutathion- Glutaraldéhyde réduit -Poly-lysine
∘ Acide thioctique-Poly-lysine.
∘ Biotine-Poly-lysine
∘ Acide pantothénique-Poly-lysine
∘ Acide ascorbique-Poly-lysine
∘ Alpha tocophérol-Poly-lysine
∘ Acide rétinoïque-Poly-lysine
∘ Coenzyme Q10-Poly-lysine
∘ Spermine- Anhydride Glutarique -Poly-lysine
pour son utilisation comme médicament en association avec au moins une composition comprenant au moins un polycomplexe choisi parmi les polycomplexes suivants :
∘ Un polycomplexe constitué par les composés Poly-lysine suivants :
▪ Biotine-Poly-lysine
▪ Coenzyme Q10-Poly-lysine
▪ Acide rétinoïque-Poly-lysine
▪ Acide thioctique-Poly-lysine
▪ Acide pantothénique-Poly-lysine
▪ Acide ascorbique-Poly-lysine
▪ Alpha tocophérol-Poly-lysine
▪ Glutathion- Glutaraldéhyde réduit -Poly-lysine
▪ Acide oléique-Poly-lysine
∘ Un polycomplexe constitué par les composés Poly-lysine suivants :
▪ Acide oléique-Poly-lysine
▪ Acide palmitoléique-Poly-lysine
▪ Acide linoléique-Poly-lysine
▪ Cholestérol-Poly-lysine
▪ Acide laurique-Poly-lysine
▪ Acide palmitique-Poly-lysine
▪ Acide myristique-Poly-lysine
▪ Acide thioctique-Poly-lysine
∘ Un polycomplexe constitué par les composés Poly-lysine suivants :
▪ Acide laurique-Poly-lysine
▪ Acide lactique-Poly-lysine
▪ Acide pyruvique-Poly-lysine
∘ Un polycomplexe constitué par les composés Poly-lysine suivants :
▪ Acide acétique-Poly-lysine
▪ Acide butyrique-Poly-lysine
▪ Acide lactique-Poly-lysine
▪ Acide propionique-Poly-lysine
▪ Acide pyruvique-Poly-lysine.
∘ un polycomplexe constitué par les composés Poly-lysine suivants :
▪ Acide rétinoïque-Poly-lysine
▪ Alpha tocophérol-Poly-lysine
▪ Acide ascorbique-Poly-lysine
▪ Coenzyme Q10-Poly-lysine
▪ Spermine- Anhydride Glutarique -Poly-lysine.
▪ Cystéine- Anhydride Glutarique -Poly-lysine
▪ Cystéine- Glutaraldéhyde réduit -Poly-lysine
▪ Méthionine- Anhydride Glutarique -Poly-lysine
▪ Méthionine- Glutaraldéhyde réduit -Poly-lysine
▪ Taurine- Anhydride Glutarique -Poly-lysine
▪ Taurine- Glutaraldéhyde réduit -Poly-lysine
o un polycomplexe constitué par les composés Poly-lysine suivants :
▪ Acide oléique-Poly-lysine
▪ Farnésyl cystéine-Poly-lysine
▪ Cholestérol-Poly-lysine
▪ Acide palmitoléique-Poly-lysine
▪ Acide palmitique-Poly-lysine
▪ Acide linoléique-Poly-lysine
▪ Acide myristique-Poly-lysine
▪ Acide thioctique-Poly-lysine
▪ Acide azélaïque-Poly-lysine
o un polycomplexe constitué par les composés Poly-lysine suivants :
▪ Acide oléique-Poly-lysine
▪ Acide thioctique-Poly-lysine
▪ Acide rétinoïque-Poly-lysine
▪ Coenzyme Q10-Poly-lysine
o un polycomplexe constitué par les composés Poly-lysine suivants :
▪ Acide laurique-Poly-lysine
▪ Acide oléique-Poly-lysine
▪ Acide palmitoléique-Poly-lysine
▪ Acide linoléique-Poly-lysine
▪ Cholestérol-Poly-lysine
▪ Acide palmitique-Poly-lysine
▪ Acide myristique-Poly-lysine
▪ Acide thioctique-Poly-lysine
▪ Coenzyme Q10-Poly-lysine
▪ Acide rétinoïque-Poly-lysine
▪ Acide pyruvique-Poly-lysine.
▪ Glutathion- Glutaraldéhyde réduit -Poly-lysine
▪ Cystéine- Anhydride Glutarique -Poly-lysine
▪ Cystéine- Glutaraldéhyde réduit -Poly-lysine
▪ Taurine- Anhydride Glutarique -Poly-lysine
▪ Taurine- Glutaraldéhyde réduit -Poly-lysine
▪ Méthionine- Anhydride Glutarique -Poly-lysine
▪ Méthionine- Glutaraldéhyde réduit -Poly-lysine
o un polycomplexe constitué par les composés Poly-lysine suivants :
▪ Acide oléique-Poly-lysine
▪ Acide azélaïque-Poly-lysine
▪ Farnésyl cystéine-Poly-lysine
▪ Acide thioctique-Poly-lysine
▪ Acide palmitique-Poly-lysine
▪ Méthionine- Anhydride Glutarique -Poly-lysine
▪ Taurine- Anhydride Glutarique -Poly-lysine
▪ Acide gamma-aminobutyrique- Glutaraldéhyde réduit -Poly-lysine
o un polycomplexe constitué par les composés Poly-lysine suivants :
▪ 5-hydroxytryptamine- Anhydride Glutarique -Poly-lysine
▪ 5-hydroxytryptophane- Anhydride Glutarique -Poly-lysine
▪ LDopa- Anhydride Glutarique -Poly-lysine
▪ Méthionine- Glutaraldéhyde réduit -Poly-lysine
▪ Taurine- Glutaraldéhyde réduit -Poly-lysine
▪ Acide gamma-aminobutyrique- Glutaraldéhyde réduit -Poly-lysine.
chaque polycomplexe étant constitué par plusieurs composés Poly-lysine, lesdits composés Poly-lysine étant constitués par au moins une petite molécule conjuguée à une Poly-lysine.

7. Composition pour son utilisation selon l'une des revendications 4 à 6, dans la prévention ou le traitement de la Sclérose Latérale Amyotrophique (SLA).

## Patentansprüche

1. Zusammensetzung, umfassend mindestens zwei Polykomplexe, wobei jeder Polykomplex aus mehreren Polylysinverbindungen besteht, wobei die Polylysinverbindungen aus mindestens einem kleinen Molekül bestehen, das mit einem Polylysin konjugiert ist, **dadurch gekennzeichnet, dass**
- mindestens ein Polykomplex aus den folgenden Polykomplexen ausgewählt ist:
o einem Polykomplex, der ausschließlich aus den folgenden Polylysinverbindungen besteht:
▪ Cystein-Glutarsäureanhydrid-Polylysin
▪ Cystein- reduziertes Glutaraldehyd -Polylysin
▪ Taurin-Glutarsäureanhydrid-Polylysin
▪ Taurin- reduziertes Glutaraldehyd -Polylysin
▪ Methionin-Glutarsäureanhydrid-Polylysin
▪ Methionin- reduziertes Glutaraldehyd -Polylysin
▪ Glutathion- reduziertes Glutaraldehyd -Polylysin
▪ Thioctsäure-Polylysin.
∘ einem Polykomplex, der ausschließlich aus den folgenden Polylysinverbindungen besteht:
▪ Cystein-Glutarsäureanhydrid-Polylysin
▪ Cystein- reduziertes Glutaraldehyd -Polylysin
▪ Taurin-Glutarsäureanhydrid-Polylysin
▪ Taurin- reduziertes Glutaraldehyd -Polylysin
▪ Methionin-Glutarsäureanhydrid-Polylysin
▪ Methionin- reduziertes Glutaraldehyd -Polylysin
▪ Glutathion- reduziertes Glutaraldehyd -Polylysin
▪ Thioctsäure-Polylysin.
▪ Biotin-Polylysin
▪ Pantothensäure-Polylysin
▪ Ascorbinsäure-Polylysin
▪ Alpha-Tocopherol-Polylysin
▪ Retinsäure-Polylysin
▪ Coenzym-Q10-Polylysin
▪ Spermin-Glutarsäureanhydrid-Polylysin
- und mindestens Polykomplex aus den folgenden Polykomplexen ausgewählt ist:
o einem Polykomplex, der aus den folgenden Polylysinverbindungen besteht:
▪ Biotin-Polylysin
▪ Coenzym-Q10-Polylysin
▪ Retinsäure-Polylysin
▪ Thioctsäure-Polylysin
▪ Pantothensäure-Polylysin
▪ Ascorbinsäure-Polylysin
▪ Alpha-Tocopherol-Polylysin
▪ Glutathion- reduziertes Glutaraldehyd -Polylysin
▪ Ölsäure-Polylysin
o einem Polykomplex, der aus den folgenden Polylysinverbindungen besteht:
▪ Ölsäure-Polylysin
▪ Palmitoleinsäure-Polylysin
▪ Linolsäure-Polylysin
▪ Cholesterin-Polylysin
▪ Laurinsäure-Polylysin
▪ Palmitinsäure-Polylysin
▪ Myristinsäure-Polylysin
▪ Thioctsäure-Polylysin
o einem Polykomplex, der aus den folgenden Polylysinverbindungen besteht:
▪ Laurinsäure-Polylysin
▪ Milchsäure-Polylysin
▪ Brenztraubensäure-Polylysin
o einem Polykomplex, der aus den folgenden Polylysinverbindungen besteht:
▪ Essigsäure-Polylysin
▪ Buttersäure-Polylysin
▪ Milchsäure-Polylysin
▪ Propionsäure-Polylysin
▪ Brenztraubensäure-Polylysin
o einem Polykomplex, der aus den folgenden Polylysinverbindungen besteht:
▪ Retinsäure-Polylysin
▪ Alpha-Tocopherol-Polylysin
▪ Ascorbinsäure-Polylysin
▪ Coenzym-Q10-Polylysin
▪ Spermin-Glutarsäureanhydrid-Polylysin
▪ Cystein-Glutarsäureanhydrid-Polylysin
▪ Cystein- reduziertes Glutaraldehyd -Polylysin
▪ Methionin-Glutarsäureanhydrid-Polylysin
▪ Methionin- reduziertes Glutaraldehyd -Polylysin
▪ Taurin-Glutarsäureanhydrid-Polylysin
▪ Taurin- reduziertes Glutaraldehyd -Polylysin
o einem Polykomplex, der aus den folgenden Polylysinverbindungen besteht:
▪ Ölsäure-Polylysin
▪ Farnesylcystein-Polylysin
▪ Cholesterin-Polylysin
▪ Palmitoleinsäure-Polylysin
▪ Palmitinsäure-Polylysin
▪ Linolsäure-Polylysin
▪ Myristinsäure-Polylysin
▪ Thioctsäure-Polylysin
▪ Azelainsäure-Polylysin
o einem Polykomplex, der aus den folgenden Polylysinverbindungen besteht:
▪ Ölsäure-Polylysin
▪ Thioctsäure-Polylysin
▪ Retinsäure-Polylysin
▪ Coenzym-Q10-Polylysin
o einem Polykomplex, der aus den folgenden Polylysinverbindungen besteht:
▪ Laurinsäure-Polylysin
▪ Ölsäure-Polylysin
▪ Palmitoleinsäure-Polylysin
▪ Linolsäure-Polylysin
▪ Cholesterin-Polylysin
▪ Palmitinsäure-Polylysin
▪ Myristinsäure-Polylysin
▪ Thioctsäure-Polylysin
▪ Coenzym-Q10-Polylysin
▪ Retinsäure-Polylysin
▪ Brenztraubensäure-Polylysin.
▪ Glutathion- reduziertes Glutaraldehyd -Polylysin
▪ Cystein-Glutarsäureanhydrid-Polylysin
▪ Cystein- reduziertes Glutaraldehyd -Polylysin
▪ Taurin-Glutarsäureanhydrid -Polylysin
▪ Taurin- reduziertes Glutaraldehyd -Polylysin
▪ Methionin-Glutarsäureanhydrid-Polylysin
▪ Methionin- reduziertes Glutaraldehyd -Polylysin
o einem Polykomplex, der aus den folgenden Polylysinverbindungen besteht:
▪ Ölsäure-Polylysin
▪ Azelainsäure-Polylysin
▪ Farnesylcystein-Polylysin
▪ Thioctsäure-Polylysin
▪ Palmitinsäure-Polylysin
▪ Methionin- Glutarsäureanhydrid -Polylysin
▪ Taurin-Glutarsäureanhydrid -Polylysin
▪ Gamma-Aminobuttersäure- reduziertes Glutaraldehyd -Polylysin
o einem Polykomplex, der aus den folgenden Polylysinverbindungen besteht:
▪ 5-Hydroxytryptamin-Glutarsäureanhydrid-Polylysin
▪ 5-Hydroxytryptophan-Glutarsäureanhydrid-Polylysin
▪ LDopa-Glutarsäureanhydrid-Polylysin
▪ Methionin- reduziertes Glutaraldehyd -Polylysin
▪ Taurin- reduziertes Glutaraldehyd -Polylysin
▪ Gamma-Aminobuttersäure- reduziertes Glutaraldehyd -Polylysin.

2. Zusammensetzung nach dem vorstehenden Anspruch,
**dadurch gekennzeichnet, dass** der oder die Polykomplex(e), umfassend Antioxidantien und/oder Radikalfänger, in einer Menge vorhanden sind, die bezogen auf das Gewicht mindestens 2,5-mal größer als die anderen Polykomplexe ist.

3. Zusammensetzung nach einem der vorstehenden Ansprüche,
**dadurch gekennzeichnet, dass** die Konzentration jedes der kleinen Moleküle in jedem Polykomplex zwischen 6,10⁻⁵ M und 1,10⁻⁴ M liegt.

4. Zusammensetzung nach einem der vorstehenden Ansprüche zur Verwendung als Arzneimittel.

5. Zusammensetzung, umfassend mindestens einen Polykomplex, der ausschließlich aus den folgenden Polylysinverbindungen besteht:
o Cystein-Glutarsäureanhydrid-Polylysin
o Cystein- reduziertes Glutaraldehyd -Polylysin
o Taurin-Glutarsäureanhydrid-Polylysin
o Taurin- reduziertes Glutaraldehyd -Polylysin
o Methionin-Glutarsäureanhydrid-Polylysin
o Methionin- reduziertes Glutaraldehyd -Polylysin
o Glutathion- reduziertes Glutaraldehyd -Polylysin
o Thioctsäure-Polylysin,
zur Verwendung als Arzneimittel in Verbindung mit mindestens einer Zusammensetzung, umfassend mindestens einen Polykomplex, der aus den folgenden Polykomplexen ausgewählt ist:
o einem Polykomplex, der aus den folgenden Polylysinverbindungen besteht:
▪ Biotin-Polylysin
▪ Coenzym-Q10-Polylysin
▪ Retinsäure-Polylysin
▪ Thiocinsäure-Polylysin
▪ Pantothensäure-Polylysin
▪ Ascorbinsäure-Polylysin
▪ Alpha-Tocopherol-Polylysin
▪ Glutathion- reduziertes Glutaraldehyd -Polylysin
▪ Ölsäure-Polylysin
o einem Polykomplex, der aus den folgenden Polylysinverbindungen besteht:
▪ Ölsäure-Polylysin
▪ Palmitoleinsäure-Polylysin
▪ Linolsäure-Polylysin
▪ Cholesterin-Polylysin
▪ Laurinsäure-Polylysin
▪ Palmitinsäure-Polylysin
▪ Myristinsäure-Polylysin
▪ Thiocinsäure-Polylysin
o einem Polykomplex, der aus den folgenden Polylysinverbindungen besteht:
▪ Laurinsäure-Polylysin
▪ Milchsäure-Polylysin
▪ Brenztraubensäure-Polylysin
o einem Polykomplex, der aus den folgenden Polylysinverbindungen besteht:
▪ Essigsäure-Polylysin
▪ Buttersäure-Polylysin
▪ Milchsäure-Polylysin
▪ Propionsäure-Polylysin
▪ Brenztraubensäure-Polylysin
o einem Polykomplex, der aus den folgenden Polylysinverbindungen besteht:
▪ Retinsäure-Polylysin
▪ Alpha-Tocopherol-Polylysin
▪ Ascorbinsäure-Polylysin
▪ Coenzym-Q10-Polylysin
▪ Spermin-Glutarsäureanhydrid-Polylysin.
▪ Cystein-Glutarsäureanhydrid-Polylysin
▪ Cystein- reduziertes Glutaraldehyd -Polylysin
▪ Methionin-Glutarsäureanhydrid-Polylysin
▪ Methionin- reduziertes Glutaraldehyd -Polylysin
▪ Taurin-Glutarsäureanhydrid-Polylysin
▪ Taurin- reduziertes Glutaraldehyd -Polylysin
o einem Polykomplex, der aus den folgenden Polylysinverbindungen besteht:
▪ Ölsäure-Polylysin
▪ Farnesyl-Cystein-Polylysin
▪ Cholesterin-Polylysin
▪ Palmitoleinsäure-Polylysin
▪ Palmitinsäure-Polylysin
▪ Linolsäure-Polylysin
▪ Myristinsäure-Polylysin
▪ Thiocinsäure-Polylysin
▪ Azelainsäure-Polylysin
o einem Polykomplex, der aus den folgenden Polylysinverbindungen besteht:
▪ Ölsäure-Polylysin
▪ Thiocinsäure-Polylysin
▪ Retinsäure-Polylysin
▪ Coenzym-Q10-Polylysin.
wobei jeder Polykomplex aus mehreren Polylysinverbindungen besteht, wobei die Polylysinverbindungen aus mindestens einem kleinen Molekül bestehen, das mit einem Polylysin konjugiert ist.

6. Zusammensetzung, umfassend mindestens einen Polykomplex, der ausschließlich aus den folgenden Polylysinverbindungen besteht:
o Cystein-Glutarsäureanhydrid-Polylysin
o Cystein- reduziertes Glutaraldehyd -Polylysin
o Taurin-Glutarsäureanhydrid-Polylysin
o Taurin- reduziertes Glutaraldehyd -Polylysin
o Methionin-Glutarsäureanhydrid-Polylysin
∘ Methionin- reduziertes Glutaraldehyd -Polylysin
∘ Glutathion- reduziertes Glutaraldehyd -Polylysin
∘ Thioctsäure-Polylysin.
∘ Biotin-Polylysin
∘ Pantothensäure-Polylysin
∘ Ascorbinsäure-Polylysin
∘ Alpha-Tocopherol-Polylysin
∘ Retinsäure-Polylysin
∘ Coenzym-Q10-Polylysin
∘ Spermin-Glutarsäureanhydrid-Polylysin
zur Verwendung als Arzneimittel in Verbindung mit mindestens einer Zusammensetzung, umfassend mindestens einen Polykomplex, der aus den folgenden Polykomplexen ausgewählt ist:
o einem Polykomplex, der aus den folgenden Polylysinverbindungen besteht:
▪ Biotin-Polylysin
▪ Coenzym-Q10-Polylysin
▪ Retinsäure-Polylysin
▪ Thioctsäure-Polylysin
▪ Pantothensäure-Polylysin
▪ Ascorbinsäure-Polylysin
▪ Alpha-Tocopherol-Polylysin
▪ Glutathion- reduziertes Glutaraldehyd -Polylysin
▪ Ölsäure-Polylysin
o einem Polykomplex, der aus den folgenden Polylysinverbindungen besteht:
▪ Ölsäure-Polylysin
▪ Palmitoleinsäure-Polylysin
▪ Linolsäure-Polylysin
▪ Cholesterin-Polylysin
▪ Laurinsäure-Polylysin
▪ Palmitinsäure-Polylysin
▪ Myristinsäure-Polylysin
▪ Thioctsäure-Polylysin
o einem Polykomplex, der aus den folgenden Polylysinverbindungen besteht:
▪ Laurinsäure-Polylysin
▪ Milchsäure-Polylysin
▪ Brenztraubensäure-Polylysin
o einem Polykomplex, der aus den folgenden Polylysinverbindungen besteht:
▪ Essigsäure-Polylysin
▪ Buttersäure-Polylysin
▪ Milchsäure-Polylysin
▪ Propionsäure-Polylysin
▪ Brenztraubensäure-Polylysin.
∘ einem Polykomplex, der aus den folgenden Polylysinverbindungen besteht:
▪ Retinsäure-Polylysin
▪ Alpha-Tocopherol-Polylysin
▪ Ascorbinsäure-Polylysin
▪ Coenzym-Q10-Polylysin
▪ Spermin-Glutarsäureanhydrid-Polylysin.
▪ Cystein-Glutarsäureanhydrid-Polylysin
▪ Cystein- reduziertes Glutaraldehyd -Polylysin
▪ Methionin-Glutarsäureanhydrid-Polylysin
▪ Methionin- reduziertes Glutaraldehyd -Polylysin
▪ Taurin-Glutarsäureanhydrid-Polylysin
▪ Taurin- reduziertes Glutaraldehyd -Polylysin
o einem Polykomplex, der aus den folgenden Polylysinverbindungen besteht:
▪ Ölsäure-Polylysin
▪ Farnesylcystein-Polylysin
▪ Cholesterin-Polylysin
▪ Palmitoleinsäure-Polylysin
▪ Palmitinsäure-Polylysin
▪ Linolsäure-Polylysin
▪ Myristinsäure-Polylysin
▪ Thioctsäure-Polylysin
▪ Azelainsäure-Polylysin
o einem Polykomplex, der aus den folgenden Polylysinverbindungen besteht:
▪ Ölsäure-Polylysin
▪ Thioctsäure-Polylysin
▪ Retinsäure-Polylysin
▪ Coenzym-Q10-Polylysin
o einem Polykomplex, der aus den folgenden Polylysinverbindungen besteht:
▪ Laurinsäure-Polylysin
▪ Ölsäure-Polylysin
▪ Palmitoleinsäure-Polylysin
▪ Linolsäure-Polylysin
▪ Cholesterin-Polylysin
▪ Palmitinsäure-Polylysin
▪ Myristinsäure-Polylysin
▪ Thioctsäure-Polylysin
▪ Coenzym-Q10-Polylysin
▪ Retinsäure-Polylysin
▪ Brenztraubensäure-Polylysin.
▪ Glutathion- reduziertes Glutaraldehyd -Polylysin
▪ Cystein-Glutarsäureanhydrid-Polylysin
▪ Cystein- reduziertes Glutaraldehyd -Polylysin
▪ Taurin-Glutarsäureanhydrid-Polylysin
▪ Taurin- reduziertes Glutaraldehyd -Polylysin
▪ Methionin-Glutarsäureanhydrid-Polylysin
▪ Methionin- reduziertes Glutaraldehyd -Polylysin
o einem Polykomplex, der aus den folgenden Polylysinverbindungen besteht:
▪ Ölsäure-Polylysin
▪ Azelainsäure-Polylysin
▪ Farnesylcystein-Polylysin
▪ Thioctsäure-Polylysin
▪ Palmitinsäure-Polylysin
▪ Methionin-Glutarsäureanhydrid-Polylysin
▪ Taurin-Glutarsäureanhydrid-Polylysin
▪ Gamma-Aminobuttersäure- reduziertes Glutaraldehyd -Polylysin
o einem Polykomplex, der aus den folgenden Polylysinverbindungen besteht:
▪ 5-Hydroxytryptamin-Glutarsäureanhydrid-Polylysin
▪ 5-Hydroxytryptophan-Glutarsäureanhydrid-Polylysin
▪ LDopa- Glutarsäureanhydrid-Polylysin
▪ Methionin- reduziertes Glutaraldehyd -Polylysin
▪ Taurin- reduziertes Glutaraldehyd -Polylysin
▪ Gamma-Aminobuttersäure- reduziertes Glutaraldehyd -Polylysin.
wobei jeder Polykomplex aus mehreren Polylysinverbindungen besteht, wobei die Polylysinverbindungen aus mindestens einem kleinen Molekül bestehen, das mit einem Polylysin konjugiert ist.

7. Zusammensetzung zur Verwendung nach einem der Ansprüche 4 bis 6 für die Prävention oder die Behandlung von amyotrophischer Lateralsklerose (ALS).

## Claims

1. Composition comprising at least two polycomplexes, each polycomplex consisting of a plurality of polylysine compounds, said polylysine compounds consisting of at least one small molecule conjugated to a polylysine,
**characterized in that**
- at least one polycomplex is selected from the following polycomplexes:
∘ a polycomplex consisting exclusively of the following polylysine compounds:
▪ cysteine-glutaric anhydride polylysine
▪ cysteine-reduced glutaraldehyde polylysine
▪ taurine-glutaric anhydride polylysine
▪ taurine-reduced glutaraldehyde polylysine
▪ methionine-glutaric anhydride polylysine
▪ methionine-reduced glutaraldehyde polylysine
▪ glutathione-reduced glutaraldehyde polylysine
▪ thioctic acid polylysine
∘ a polycomplex consisting exclusively of the following polylysine compounds:
▪ cysteine-glutaric anhydride polylysine
▪ cysteine-reduced glutaraldehyde polylysine
▪ taurine-glutaric anhydride polylysine
▪ taurine-reduced glutaraldehyde polylysine
▪ methionine-glutaric anhydride polylysine
▪ methionine-reduced glutaraldehyde polylysine
▪ glutathione-reduced glutaraldehyde polylysine
▪ thioctic acid polylysine
▪ biotin polylysine
▪ pantothenic acid polylysine
▪ ascorbic acid polylysine
▪ alpha tocopherol polylysine
▪ retinoic acid polylysine
▪ coenzyme Q10 polylysine
▪ spermine-glutaric anhydride polylysine
- and at least one polycomplex is selected from the following polycomplexes:
∘ a polycomplex consisting of the following polylysine compounds:
▪ biotin polylysine
▪ coenzyme Q10 polylysine
▪ retinoic acid polylysine
▪ thioctic acid polylysine
▪ pantothenic acid polylysine
▪ ascorbic acid polylysine
▪ alpha tocopherol polylysine
▪ glutathione-reduced glutaraldehyde polylysine
▪ oleic acid polylysine
∘ a polycomplex consisting of the following polylysine compounds:
▪ oleic acid polylysine
▪ palmitoleic acid polylysine
▪ linoleic acid polylysine
▪ cholesterol polylysine
▪ lauric acid polylysine
▪ palmitic acid polylysine
▪ myristic acid polylysine
▪ thioctic acid polylysine
∘ a polycomplex consisting of the following polylysine compounds:
▪ lauric acid polylysine
▪ lactic acid polylysine
▪ pyruvic acid polylysine
∘ a polycomplex consisting of the following polylysine compounds:
▪ acetic acid polylysine
▪ butyric acid polylysine
▪ lactic acid polylysine
▪ propionic acid polylysine
▪ pyruvic acid polylysine
∘ a polycomplex consisting of the following polylysine compounds:
▪ retinoic acid polylysine
▪ alpha tocopherol polylysine
▪ ascorbic acid polylysine
▪ coenzyme Q10 polylysine
▪ spermine-glutaric anhydride polylysine
▪ cysteine-glutaric anhydride polylysine
▪ cysteine-reduced glutaraldehyde polylysine
▪ methionine-glutaric anhydride polylysine
▪ methionine-reduced glutaraldehyde polylysine
▪ taurine-glutaric anhydride polylysine
▪ taurine-reduced glutaraldehyde polylysine
∘ a polycomplex consisting of the following polylysine compounds:
▪ oleic acid polylysine
▪ farnesyl cysteine polylysine
▪ cholesterol polylysine
▪ palmitoleic acid polylysine
▪ palmitic acid polylysine
▪ linoleic acid polylysine
▪ myristic acid polylysine
▪ thioctic acid polylysine
▪ azelaic acid polylysine
∘ a polycomplex consisting of the following polylysine compounds:
▪ oleic acid polylysine
▪ thioctic acid polylysine
▪ retinoic acid polylysine
▪ coenzyme Q10 polylysine
∘ a polycomplex consisting of the following polylysine compounds:
▪ lauric acid polylysine
▪ oleic acid polylysine
▪ palmitoleic acid polylysine
▪ linoleic acid polylysine
▪ cholesterol polylysine
▪ palmitic acid polylysine
▪ myristic acid polylysine
▪ thioctic acid polylysine
▪ coenzyme Q10 polylysine
▪ retinoic acid polylysine
▪ pyruvic acid polylysine
▪ glutathione-reduced glutaraldehyde polylysine
▪ cysteine-glutaric anhydride polylysine
▪ cysteine-reduced glutaraldehyde polylysine
▪ taurine-glutaric anhydride polylysine
▪ taurine-reduced glutaraldehyde polylysine
▪ methionine-glutaric anhydride polylysine
▪ methionine-reduced glutaraldehyde polylysine
∘ a polycomplex consisting of the following polylysine compounds:
▪ oleic acid polylysine
▪ azelaic acid polylysine
▪ farnesyl cysteine polylysine
▪ thioctic acid polylysine
▪ palmitic acid polylysine
▪ methionine-glutaric anhydride polylysine
▪ taurine-glutaric anhydride polylysine
▪ gamma-aminobutyric-acid-reduced glutaraldehyde polylysine
∘ a polycomplex consisting of the following polylysine compounds:
▪ 5-hydroxytryptamine-glutaric anhydride polylysine
▪ 5-hydroxytryptophane-glutaric anhydride polylysine
▪ L-dopa-glutaric anhydride polylysine
▪ methionine-reduced glutaraldehyde polylysine
▪ taurine-reduced glutaraldehyde polylysine
▪ gamma-aminobutyric-acid-reduced glutaraldehyde polylysine.

2. Composition according to the preceding claim, **characterized in that** the polycomplex(es) comprising antioxidants and/or free radical scavengers are present in an amount at least 2.5 times greater by weight than the other polycomplexes.

3. Composition according to either of the preceding claims,
**characterized in that** the concentration of each of said small molecules in each polycomplex is between 6.10⁻⁵ M and 1.10⁻⁴ M.

4. Composition according to any of the preceding claims for use as a medicament.

5. Composition comprising at least one polycomplex consisting exclusively of the following polylysine compounds:
∘ cysteine-glutaric anhydride polylysine
∘ cysteine-reduced glutaraldehyde polylysine
∘ taurine-glutaric anhydride polylysine
∘ taurine-reduced glutaraldehyde polylysine
∘ methionine-glutaric anhydride polylysine
∘ methionine-reduced glutaraldehyde polylysine
∘ glutathione-reduced glutaraldehyde polylysine
∘ thioctic acid polylysine
for use as a medicament in combination with at least one composition comprising at least one polycomplex selected from the following polycomplexes:
∘ a polycomplex consisting of the following polylysine compounds:
▪ biotin polylysine
▪ coenzyme Q10 polylysine
▪ retinoic acid polylysine
▪ thioctic acid polylysine
▪ pantothenic acid polylysine
▪ ascorbic acid polylysine
▪ alpha tocopherol polylysine
▪ glutathione-reduced glutaraldehyde polylysine
▪ oleic acid polylysine
∘ a polycomplex consisting of the following polylysine compounds:
▪ oleic acid polylysine
▪ palmitoleic acid polylysine
▪ linoleic acid polylysine
▪ cholesterol polylysine
▪ lauric acid polylysine
▪ palmitic acid polylysine
▪ myristic acid polylysine
▪ thioctic acid polylysine
∘ a polycomplex consisting of the following polylysine compounds:
▪ lauric acid polylysine
▪ lactic acid polylysine
▪ pyruvic acid polylysine
∘ a polycomplex consisting of the following polylysine compounds:
▪ acetic acid polylysine
▪ butyric acid polylysine
▪ lactic acid polylysine
▪ propionic acid polylysine
▪ pyruvic acid polylysine
∘ a polycomplex consisting of the following polylysine compounds:
▪ retinoic acid polylysine
▪ alpha tocopherol polylysine
▪ ascorbic acid polylysine
▪ coenzyme Q10 polylysine
▪ spermine-glutaric anhydride polylysine
▪ cysteine-glutaric anhydride polylysine
▪ cysteine-reduced glutaraldehyde polylysine
▪ methionine-glutaric anhydride polylysine
▪ methionine-reduced glutaraldehyde polylysine
▪ taurine-glutaric anhydride polylysine
▪ taurine-reduced glutaraldehyde polylysine
∘ a polycomplex consisting of the following polylysine compounds:
▪ oleic acid polylysine
▪ farnesyl cysteine polylysine
▪ cholesterol polylysine
▪ palmitoleic acid polylysine
▪ palmitic acid polylysine
▪ linoleic acid polylysine
▪ myristic acid polylysine
▪ thioctic acid polylysine
▪ azelaic acid polylysine
∘ a polycomplex consisting of the following polylysine compounds:
▪ oleic acid polylysine
▪ thioctic acid polylysine
▪ retinoic acid polylysine
▪ coenzyme Q10 polylysine,
each polycomplex consisting of a plurality of polylysine compounds, said polylysine compounds consisting of at least one small molecule conjugated to a polylysine.

6. Composition comprising at least one polycomplex consisting exclusively of the following polylysine compounds:
∘ cysteine-glutaric anhydride polylysine
∘ cysteine-reduced glutaraldehyde polylysine
∘ taurine-glutaric anhydride polylysine
∘ taurine-reduced glutaraldehyde polylysine
∘ methionine-glutaric anhydride polylysine
∘ methionine-reduced glutaraldehyde polylysine
∘ glutathione-reduced glutaraldehyde polylysine
∘ thioctic acid polylysine
∘ biotin polylysine
∘ pantothenic acid polylysine
∘ ascorbic acid polylysine
∘ alpha tocopherol polylysine
∘ retinoic acid polylysine
∘ coenzyme Q10 polylysine
∘ spermine-glutaric anhydride polylysine
for use as a medicament in combination with at least one composition comprising at least one polycomplex selected from the following polycomplexes:
∘ a polycomplex consisting of the following polylysine compounds:
▪ biotin polylysine
▪ coenzyme Q10 polylysine
▪ retinoic acid polylysine
▪ thioctic acid polylysine
▪ pantothenic acid polylysine
▪ ascorbic acid polylysine
▪ alpha tocopherol polylysine
▪ glutathione-reduced glutaraldehyde polylysine
▪ oleic acid polylysine
∘ a polycomplex consisting of the following polylysine compounds:
▪ oleic acid polylysine
▪ palmitoleic acid polylysine
▪ linoleic acid polylysine
▪ cholesterol polylysine
▪ lauric acid polylysine
▪ palmitic acid polylysine
▪ myristic acid polylysine
▪ thioctic acid polylysine
∘ a polycomplex consisting of the following polylysine compounds:
▪ lauric acid polylysine
▪ lactic acid polylysine
▪ pyruvic acid polylysine
∘ a polycomplex consisting of the following polylysine compounds:
▪ acetic acid polylysine
▪ butyric acid polylysine
▪ lactic acid polylysine
▪ propionic acid polylysine
▪ pyruvic acid polylysine
∘ a polycomplex consisting of the following polylysine compounds:
▪ retinoic acid polylysine
▪ alpha tocopherol polylysine
▪ ascorbic acid polylysine
▪ coenzyme Q10 polylysine
▪ spermine-glutaric anhydride polylysine
▪ cysteine-glutaric anhydride polylysine
▪ cysteine-reduced glutaraldehyde polylysine
▪ methionine-glutaric anhydride polylysine
▪ methionine-reduced glutaraldehyde polylysine
▪ taurine-glutaric anhydride polylysine
▪ taurine-reduced glutaraldehyde polylysine
∘ a polycomplex consisting of the following polylysine compounds:
▪ oleic acid polylysine
▪ farnesyl cysteine polylysine
▪ cholesterol polylysine
▪ palmitoleic acid polylysine
▪ palmitic acid polylysine
▪ linoleic acid polylysine
▪ myristic acid polylysine
▪ thioctic acid polylysine
▪ azelaic acid polylysine
∘ a polycomplex consisting of the following polylysine compounds:
▪ oleic acid polylysine
▪ thioctic acid polylysine
▪ retinoic acid polylysine
▪ coenzyme Q10 polylysine
∘ a polycomplex consisting of the following polylysine compounds:
▪ lauric acid polylysine
▪ oleic acid polylysine
▪ palmitoleic acid polylysine
▪ linoleic acid polylysine
▪ cholesterol polylysine
▪ palmitic acid polylysine
▪ myristic acid-polylysine
▪ thioctic acid polylysine
▪ coenzyme Q10 polylysine
▪ retinoic acid polylysine
▪ pyruvic acid polylysine
▪ glutathione-reduced glutaraldehyde polylysine
▪ cysteine-glutaric anhydride polylysine
▪ cysteine-reduced glutaraldehyde polylysine
▪ taurine-glutaric anhydride polylysine
▪ taurine-reduced glutaraldehyde polylysine
▪ methionine-glutaric anhydride polylysine
▪ methionine-reduced glutaraldehyde polylysine
∘ a polycomplex consisting of the following polylysine compounds:
▪ oleic acid polylysine
▪ azelaic acid polylysine
▪ farnesyl cysteine polylysine
▪ thioctic acid polylysine
▪ palmitic acid polylysine
▪ methionine-glutaric anhydride polylysine
▪ taurine-glutaric anhydride polylysine
▪ gamma-aminobutyric-acid-reduced glutaraldehyde polylysine
∘ a polycomplex consisting of the following polylysine compounds:
▪ 5-hydroxytryptamine-glutaric anhydride polylysine
▪ 5-hydroxytryptophane-glutaric anhydride polylysine
▪ L-dopa-glutaric anhydride polylysine
▪ methionine-reduced glutaraldehyde polylysine
▪ taurine-reduced glutaraldehyde polylysine
▪ gamma-aminobutyric-acid-reduced glutaraldehyde polylysine,
each polycomplex consisting of a plurality of polylysine compounds, said polylysine compounds consisting of at least one small molecule conjugated to a polylysine.

7. Composition for use according to any of claims 4 to 6 in the prevention or treatment of amyotrophic lateral sclerosis (ALS).
